# EUROPEAN PATENT SPECIFICATION

(11) **EP 3 397 753 B1**
(45) Date of publication and mention of the grant of the patent: **27.10.2021**
(21) Application number: 16826884.5
(22) Date of filing: 29.12.2016
(51) Int. Cl.: C12N 5/077, C12N 5/074, C12N 5/071, A61P 1/16

(54) **MICROTISSUE FORMATION USING STEM CELL-DERIVED HUMAN HEPATOCYTES**
BILDUNG VON MIKROGEWEBE MIT STAMMZELLEN AUS MENSCHLICHEN HEPATOZYTEN
FORMATION D'UN MICROTISSU À L'AIDE D'HÉPATOCYTES HUMAINS DÉRIVÉS DE CELLULES SOUCHES

(30) Priority: 30.12.2015 US 201562273395 P
(43) Date of publication of application: 07.11.2018
(73) Proprietor: FUJIFILM Cellular Dynamics, Inc., Madison, WI 53711 (US); FUJIFILM Corporation, Tokyo 106-8620 (JP)
(72) Inventor: HANCOCK, Michael, Kenneth, Madison, WI 53711 (US); CARLSON, Coby, Bennett, Madison, WI 53711 (US); MANN, David, A., Madison, WI 53711 (US); MURAKAMI, Yuta, Ashigarakamigun 2588577 (JP)
(74) Representative: Hoffmann Eitle
(86) International application number: PCT/US2016/069081
(87) International publication number: WO 2017/117333

(56) References cited:
- WO-A1-2009/092092
- WO-A1-2011/154552
- WO-A1-2015/158777
- KAZUO TAKAYAMA ET AL: "3D spheroid culture of hESC/hiPSC-derived hepatocyte-like cells for drug toxicity testing", BIOMATERIALS, vol. 34, no. 7, 1 February 2013 (2013-02-01), pages 1781-1789, XP055098841, ISSN: 0142-9612, DOI: 10.1016/j.biomaterials.2012.11.029
- M JOSÉ GÓMEZ-LECHÓN ET AL: "Competency of different cell models to predict human hepatotoxic drugs", EXPERT OPINION ON DRUG METABOLISM & TOXICOLOGY, vol. 10, no. 11, 9 October 2014 (2014-10-09), pages 1553-1568, XP055348088, GB ISSN: 1742-5255, DOI: 10.1517/17425255.2014.967680
- RICHARD L. GIESECK III ET AL: "Maturation of Induced Pluripotent Stem Cell Derived Hepatocytes by 3D-Culture", PLOS ONE, vol. 9, no. 1, 22 January 2014 (2014-01-22), page e86372, XP055307825, DOI: 10.1371/journal.pone.0086372

## Description

### BACKGROUND OF THE INVENTION

### 1. Field of the Invention

The present invention relates generally to the field of molecular biology and medicine. More particularly, it concerns methods for producing three-dimensional (3-D) microtissue.

### 2. Description of Related Art

Currently, a typical preclinical drug safety evaluation for liver toxicity employs cell based and animal models as predictive models. Focusing specifically on *in vitro* models, a number of cancer-derived cell lines (*e.g.,* HepG2 and Huh7) can be employed in hepatotoxicity assessments, but these cells are limited in terms of their drug metabolizing functions (Clayton *et al.,* 2005). This functional liability makes these lines questionable in terms of their predictive nature for normal, healthy hepatocyte drug sensitivity in relation to toxic metabolite formation. Access to primary human hepatic tissue in the form of isolated primary human hepatocytes has significantly improved with the advent of methods to stably cryopreserve the primary isolates (LeCluyse *et al.,* 2005). However, once removed from the *in vivo* setting, these cells do not adapt well over time to the tissue culture environment. In addition, primary hepatocytes in static two dimensional (2-D) culture require an extracellular matrix protein overlay to survive and still rapidly decline in terms of their cytochrome P450 (CYP) function (Swifta *et al.,* 2010).

The ability to direct the differentiation of human pluripotent stem cells (PSCs) such as induced pluripotent stem cells and embryonic stem cells to specific lineages including hepatocytes provides access to unlimited numbers of human hepatocytes for a wide range of applications that include development of new treatments for a spectrum of diseases, the establishment of platforms for drug discovery and predictive toxicology and the creation of *in vitro* models of disease. However, there is still a lack of highly predictive *in vitro* human toxicity models due to a lack of a comprehensive predictive correlation between current *in vitro* and *in vivo* toxicity models and population wide human outcomes. To address these concerns and enable a better clinical development path for drug safety as it relates to toxicity prediction and mechanistic investigations, the use of stem cell-derived tissues has been an intense area of research interest.

Accordingly, there is a potential for pluripotent stem cell-derived hepatocytes to be developed into predictive *in vitro* models with stability and consistency. However, there is still room for improvement in this model system as it is applied to predictive hepatotoxicity. Therefore, there is a need for methods to produce 3-D microtissue from PSC-derived hepatocytes for therapeutic and research use to provide a more complete liver-like environment and molecular cues, thus increasing its predictive power.

### SUMMARY OF THE INVENTION

The invention is defined in the appended claims. Also disclosed is the following.

Embodiments of the present disclosure provide methods and compositions for producing three-dimensional (3-D) microtissue. In a first embodiment, there is provided an *in vitro* method of producing microtissue from pluripotent stem cell (PSC)-derived hepatocytes comprising culturing a cell suspension of PSC-derived hepatocytes in the presence of one or more extracellular matrix proteins or fragments thereof in a culture container having an essentially cell non-adhesive bottom to obtain microtissue. In certain aspects, the method further comprises obtaining a cell suspension of pluripotent stem cell (PSC)-derived hepatocytes. In some aspects, the method further comprises supplementing the cell suspension with one or more extracellular matrix proteins or fragments thereof.

In some aspects, the method further comprises supplementing the cell suspension of PSC-derived hepatocytes with PSC-derived macrophages. In certain aspects, the method further comprises supplementing the cell suspension of PSC-derived hepatocytes with PSC-derived endothelial cells. In some aspects, the method further comprises supplementing the cell suspension of PSC-derived hepatocytes with PSC-derived macrophages and PSC-derived endothelial cells.

In some aspects, the culturing of step (c) is about 24 to about 48 hours. In certain aspects, the PSC-derived hepatocytes are cultured in serum-free or defined media. In some aspects, the microtissue has a diameter of at least 50 µm.

In certain aspects, the culture container is an ultra-low attachment plate. In some aspects, the culture container does not comprise a scaffold or matrix overlay. In certain aspects, the cell suspension is not viscous. In some aspects, the bottom of the culture container is concave.

In some aspects, the pluripotent stem cell is human. In certain aspects, the pluripotent stem cell is an embryonic stem cell. In other aspects, the pluripotent stem cell is an induced pluripotent stem cell.

In certain aspects, the one or more extracellular matrix proteins are selected from the group consisting of laminin, collagen type IV, entactin, heparin sulfate proteoglycan, collagen type I, fibronectin, matrix extracellular phosphoglycoprotein (MEPE), nidogen-1, F-spondin, R-spondin, tenascin, testican, vitronectin, and decorin. In some aspects, the one or more extracellular matrix proteins are laminin, collagen type IV, entactin, and heparin sulfate proteoglycan. In particular aspects, the one or more extracellular matrix proteins is GELTREX®. In some aspects, the one or more extracellular matrix proteins or fragments thereof is collagen type I recombinant peptide. For example, the collagen type I recombinant peptide is CELLNEST™. In some aspects, the one or more extracellular matrix proteins are recombinant.

In some aspects, the cell suspension comprises about 5 percent to about 30 percent of the one or more extracellular matrix proteins or fragments thereof. In certain aspects, the cell suspension comprises about 10 percent to about 20 percent of the one or more extracellular matrix proteins or fragments thereof. In some aspects, the cell suspension comprises a concentration of about 0.005 mg/mL to about 0.05 mg/mL of the one or more extracellular matrix proteins or fragments thereof. In certain aspects, the cell suspension comprises a concentration of about 0.02 mg/mL to about 0.04 mg/mL of the one or more extracellular matrix proteins or fragments thereof.

In certain aspects, the PSC-derived hepatocytes are cultured in medium comprising DMEM/F12, B27 supplement, dexamethasone, and gentamicin. In particular aspects, the medium does not comprise LCAA or phenol red.

In certain aspects, step (a) comprises: (i) thawing cryopreserved PSC-derived hepatocytes; (ii) culturing the PSC-derived hepatocytes in a two-dimensional (2-D) culture; and (iii) dissociating the PSC-derived hepatocytes with a cell detachment reagent to obtain a cell suspension. In some aspects, the 2-D culture is on an adhesive surface. In certain aspects, the adhesive surface is a matrix. In some aspects, the matrix comprises at least one extracellular matrix protein. For example, the at least one extracellular matrix protein is collagen, laminin, or fibronectin. In some aspects, the cell detachment reagent is ACCUTASE®. In certain aspects, the PSC-derived hepatocytes are not dissociated into essentially single cells. In some aspects, the dissociation does not disrupt clusters of cells.

In some aspects, the microtissue has drug-induced cytochrome P450 (CYP) activity. For example, the CYP activity is determined by CYP1A2 and/or CYP3A4 induction. In some aspects, the drug-induced CYP activity of the microtissue is increased relative to the 2-D culture of PSC-derived hepatocytes. In some aspects, the increase in drug-induced CYP activity is at least 1.5 fold.

In certain aspects, the method further comprises tuning the size of the microtissue. In some aspects, tuning comprises: (i) determining the cell density of the cell suspension; and (ii) seeding the cells at a cell density of about 5,000 cells/cm² to about 250,000 cells/cm² to obtain a microtissue with a diameter of about 100 µm to about 800 µm. In some aspects, tuning increases liver-specific function of the microtissue relative to non-tuned microtissue. In certain aspects, the liver-specific function is measured by drug-induced CYP activity. In some aspects, tuning decreases cell death of internally positioned PSC-derived hepatocytes relative to non-tuned microtissue. In certain aspects, tuning increases cell viability relative to non-tuned microtissue. For example, the cell viability is quantified by measurement of ATP.

There is disclosed a 3-D microtissue comprising an aggregate of PSC-derived hepatocytes supplemented with one or more extracellular matrix proteins or fragments thereof. In some aspects, the microtissue is produced by the methods provided herein. In some aspects, the microtissue has a diameter of about 100 µm to about 800 µm. In certain aspects, the microtissue has stable inducible CYP expression.

There is disclosed a pharmaceutical composition comprising functional microtissue produced by the methods provided herein.

There is disclosed a kit comprising 3-D microtissue comprising an aggregate of PSC-derived hepatocytes supplemented with one or more extracellular matrix proteins or fragments thereof. In some aspects, the microtissue is produced by the methods provided herein. In certain aspects, the microtissue is provided on a culture container. For example, the culture container is an ultra-low attachment (ULA) plate.

Other objects, features and advantages of the present invention will become apparent from the following detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

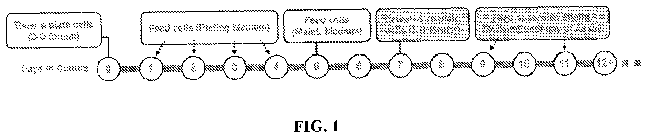

**FIG. 1****:** Schematic of 3-D microtissue formation using iCell® Hepatocytes 2.0 (stem cell-derived human hepatocytes).

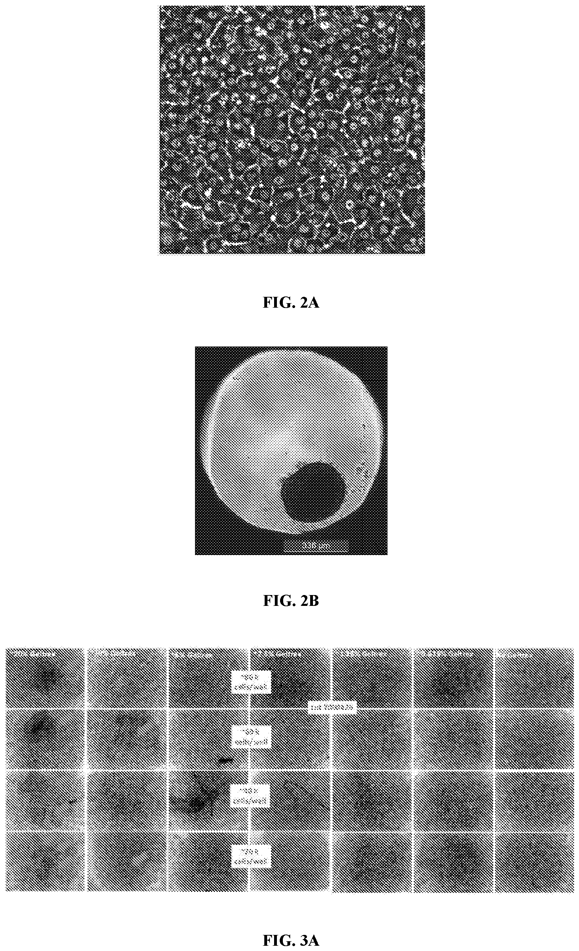

**FIGs. 2A-2B****: (A)** Image of confluent 2-D culture of iCell® Hepatocytes 2.0 (100x magnification). **(B)** Image of 3-D culture of microtissue produced from iCell® Hepatocytes 2.0 (25x magnification).
**FIGs. 3A-3E****: (A)** Screening for microtissue formation with indicated percentage of extracellular matrix supplementation to promote cell-to-cell adhesion. **(B-C)** Extracellular matrix supplementation promotes microtissue formation using thawed stem cell-derived human hepatocytes in Corning round-bottom ULA plate **(B)** or InSphero GravityTRAP ULA plate **(C). (D)** Extracellular matrix supplementation also supports microtissue formation following dissociation of cells pre-plated in 2-D format. In this example a day 12 culture of iCell® Hepatocytes 2.0 was dissociated with ACCUTASE® and then supplemented with indicated extracellular matrix, which led to spheroid formation 2 days later. **(E)** Evaluation of other materials for supplementation.

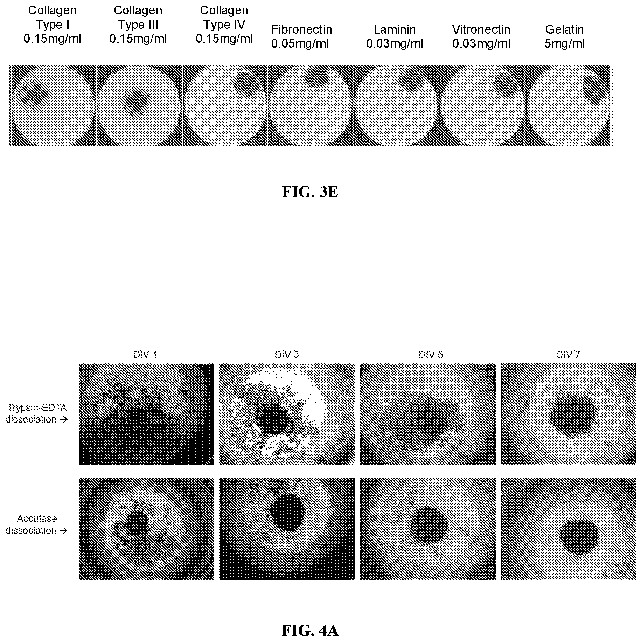

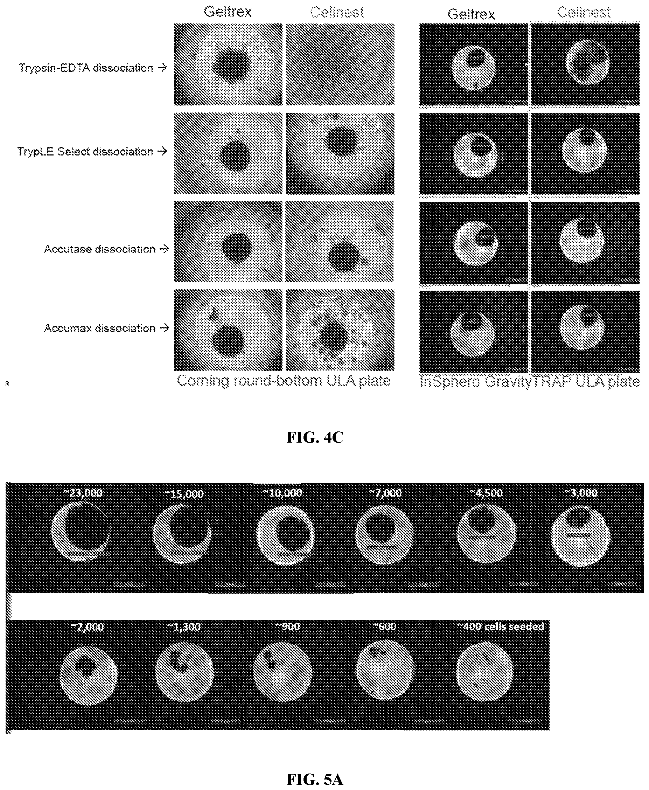

**FIGs. 4A-4C****:** Screening for optimal culture timing and dissociation conditions that combined with extracellular matrix supplementation promote efficient microtissue formation. **(A)** Images of 3-D cultures, prepared from pre-plated iCell® Hepatocyte 2.0 cultures that were dissociated by Trypsin-EDTA (top) or ACCUTASE® (bottom), taken about 48 hours after 3-D cultures were initiated. (DIV = day *in vitro,* number of days the thawed cells were pre-plated in traditional 2-D culture format prior to gentle dissociation to prepare 3-D culture). **(B)** Images of 3-D cultures prepared from 2-D cultures dissociated using one of four reagents on different days *in vitro.* Spheroid formation efficiency was affected by culture age and dissociation method, with gentler dissociation reagents (TRYPLE™ or ACCUTASE®) enabling more efficient spheroid formation at earlier timepoints. **(C)** iCell® Hepatocytes were pre-plated in 2-D format for 7 days, then dissociated with indicated dissociation agents and re-plated in 3-D culture plates for microtissue formation promoted by extracellular matrix proteins. These results indicate that spheroid formation efficiency is affected by a combination of: dissociation method (better efficiency with gentler dissociation reagents such as TRYPLE™ or ACCUTASE®), type and/or concentration of extracellular matrix supplementation used (GELTREX® was more efficient in this example than CELLNEST™), and 3-D culture plate geometry (*i.e.,* here the InSphero GravityTRAP ULA plate appeared to be more efficient than the Corning ULA plate).
**FIGs. 5A-5B****:** Microtissue size is tunable based on number of iCell® Hepatocytes supplemented with 20% of GELTREX® **(A)** or CELLNEST™ **(B)** seeded in a well of an InSphero GravityTRAP ULA plate.

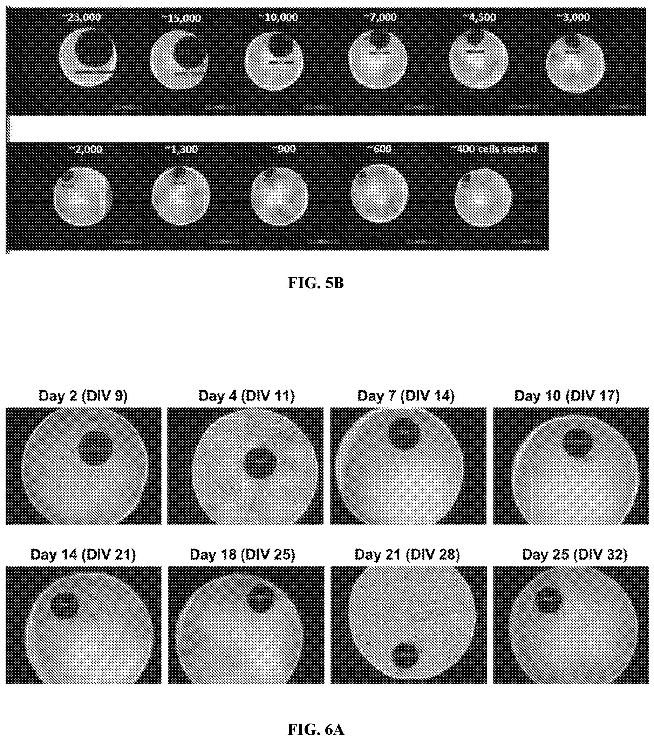

**FIGs. 6A-6B****:** Microtissue can be maintained for extended culture times. **(A)** Images taken at different days following initiation of 3-D cultures from cells that had been pre-plated for 7 days prior to dissociation, and then seeding about 2,500 cells/well supplemented with 10% GELTREX® into an InSphero GravityTRAP ULA plate. (DIV = day *in vitro,* total number of days the thawed iCell® cells were in culture, i.e., combined 3-D culture plus the initial 7-day 2-D culture time). **(B)** Graph of the average measured diameter size (n = 3 spheroids) of the 3-D microtissues over a 25 day time-course, indicating a modest decrease in size over time.

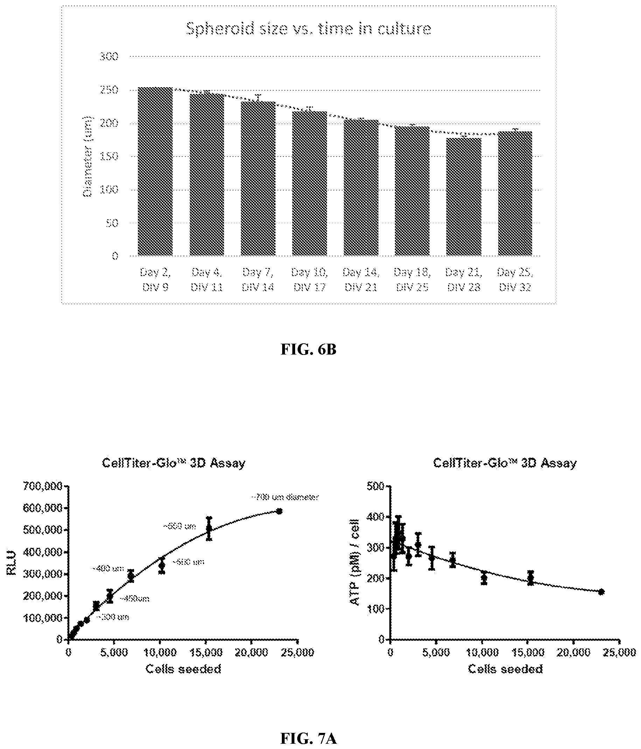

**FIGs. 7A-7C****:** Examples of functional assays that can be used to assess microtissue function and their suitability for generating predictive *in vitro* liver-like models. **(A)** ATP levels (cell viability indicator) as measured by CELLTITER-GLO® 3-D Assay for microtissue with indicated cells seeded. **(B)** Drug-induced Cytochrome P450 activity of day 6 spheroids treated with Omeprazole for 24 hours measured by P450-GLO™ CYP1A2 assay (left) or day 9 spheroids treated with Rifampicin for 96 hours measured by P450-GLO™ Cyp3A4 Assay (right). **(C)** P450-GLO™ CYP3A4 Assay of iCell® Hepatocytes in 2-D culture (left) or day 9 spheroids (right) treated for 72 hours with Rifampicin.

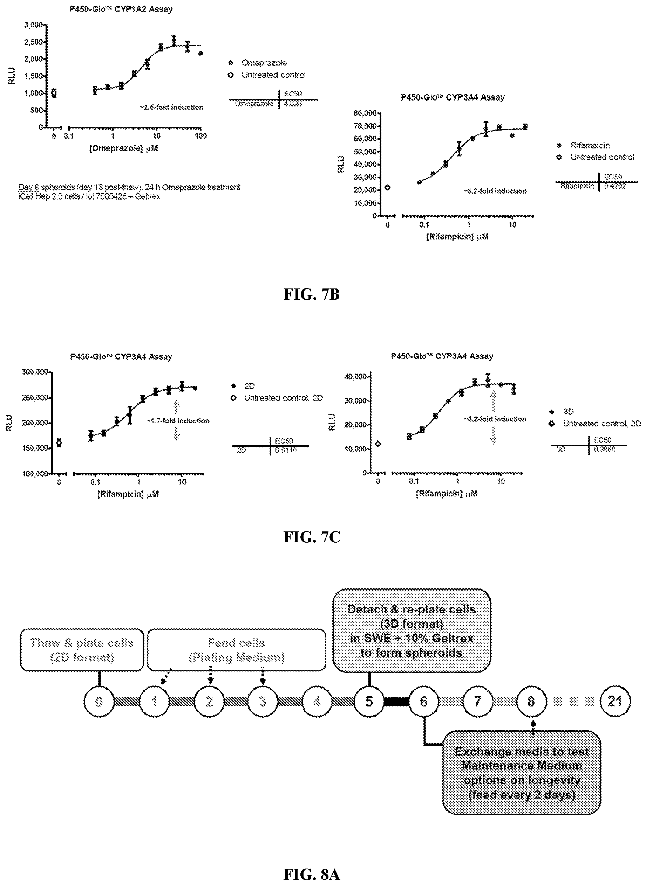

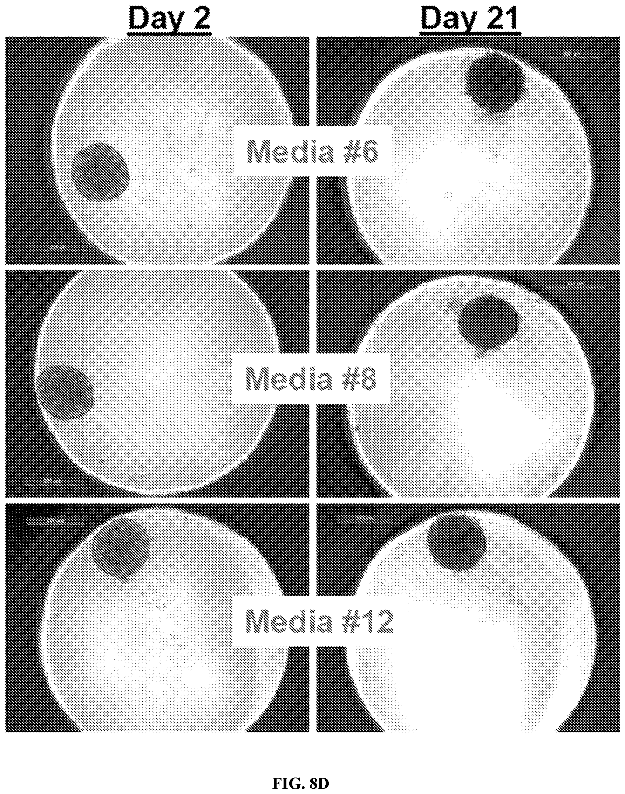

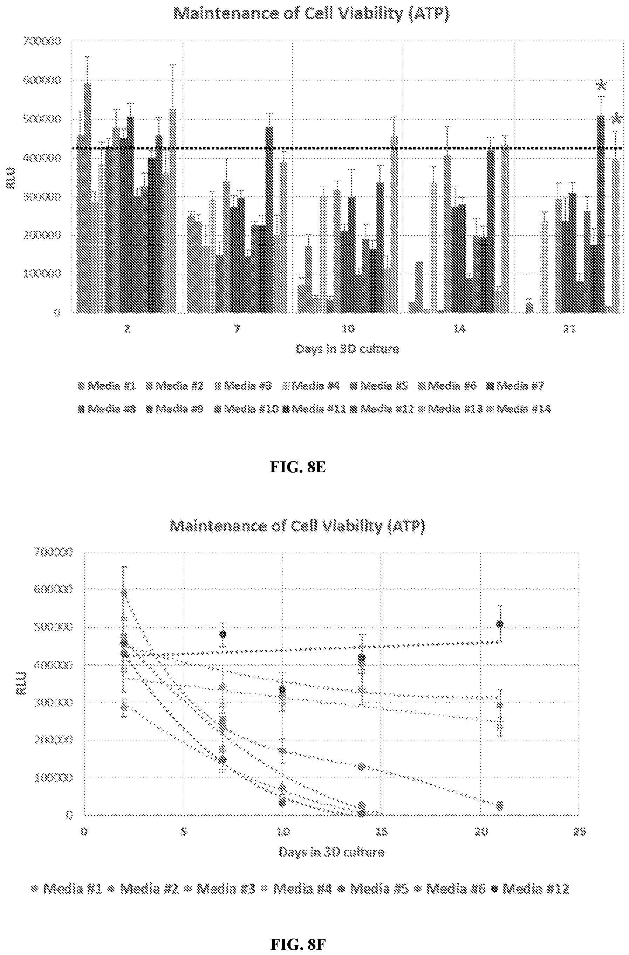

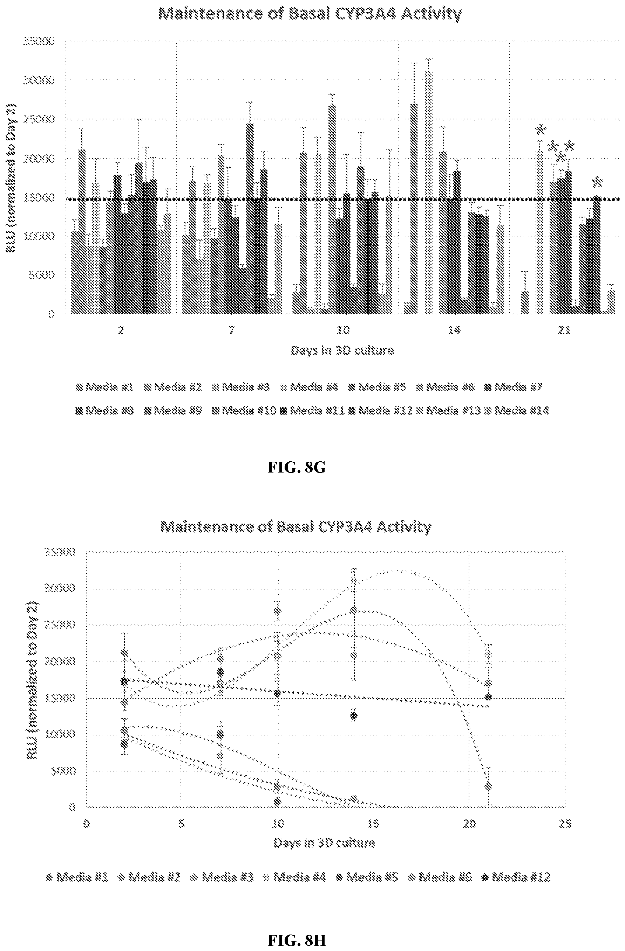

**FIGS. 8A-8H****: (A)** Schematic for testing Maintenance Medium formulations for effects on microtissue longevity. **(B)** Maintenance of spheroid morphology over 21 days in different Maintenance Medium formulations. (Asterisks denote good spheroid morphology maintenance conditions). **(C)** Graph depicting diameter (mm) of spheroids cultured in various Maintenance Media. (Left two asterisks denote inflated measurements due to precipitate collecting around spheroids; right three asterisks denote good maintenance conditions). **(D)** Images of spheroid cultures from conditions #6, #8, and #12. **(E-F)** ATP levels measured by CELLTITER-GLO ® 3D Cell Viability Assay (Promega). Media #12 had highest ATP at Day 20, followed by #6, #4, and #2. **(G-H)** CYP3A4 levels measured using the P450-GLOCYP3A4™ reagent (Promega). **(H)** At Day 20, Media #4 had the highest CYP3A4 level, followed by #6, #12, and #2. Media #1, #3, and #5 started low on Day 1 and gradually declined up to Day 15. Media #12 had a relatively consistent level around 15,000 from Day 1 to Day 20.

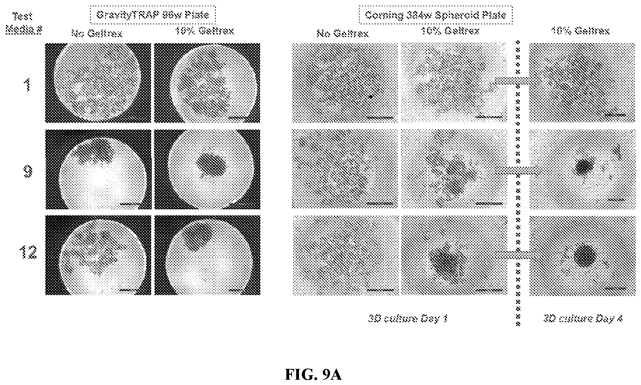

**FIGS. 9A-9C****: (A)** Spheroid formation on GravityTRAP or Corning Spheroid plates. **(B)** Spheroid formation on Corning Spheroid plates in designated media conditions. **(C)** Summary of results for media screening experiments. Media #1, #2, #3, #5, #9, and #13 had all or almost all bad results. Media #10 and #11 had average results while media #4, #6, #7, and #8 had average to good results. Media #12 had good results in all tests performed.

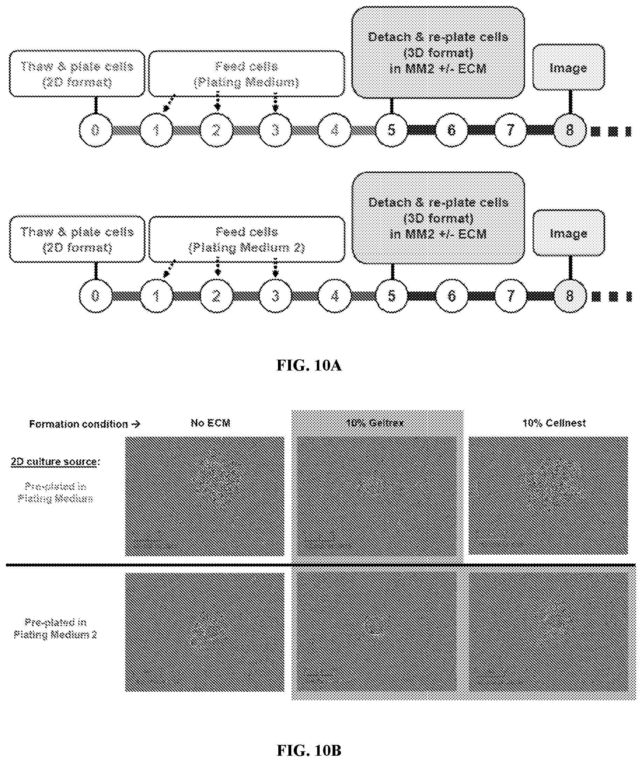

**FIGS. 10A-10G****: (A)** Schematic for characterization of 2-D Plating Medium effects on subsequent spheroid formation, longevity and functionality. **(B)** Images of spheroid formation in Corning 96-well round-bottom ULA plates using cells that had been pre-plated in 2-D format in either Plating Medium (Table 2) or Plating Medium 2 (Table 6). **(C)** Images of spheroid formation in Corning 384-well round-bottom ULA plates using cells that had been pre-plated in 2-D format in either Plating Medium (Table 2) or Plating Medium #2 (Table 6). **(D)** Images of spheroid formation in GravityTRAP ULA plates using cells that had been pre-plated in 2-D format in either Plating Medium (Table 2) or Plating Medium #2 (Table 6). **(E)** Images of spheroid formation to test longevity using cells that had been pre-plated in 2-D format in either plating media formulation. **(F)** Images of spheroid formation (in the presence of 10% GELTREX®) to test longevity using cells that had been pre-plated in 2-D format in either plating media formulation. **(G)** Functional testing measuring basal CYP3A4 activity using cells that had been pre-plated in 2-D format in either Plating Medium (PM) or Plating Medium 2 (PM2) with spheroid formation performed in the presence of 10% GELTREX® (GX) or 10% CELLNEST™ (CN).

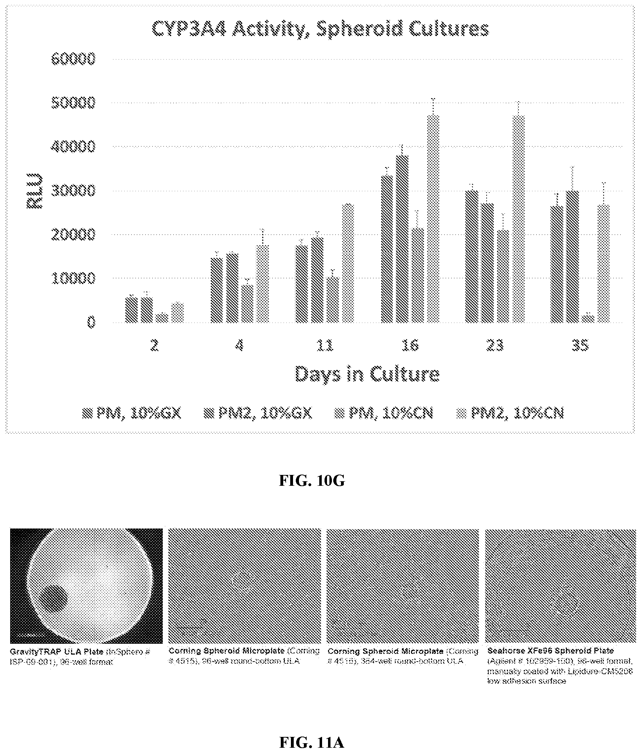

**FIGS. 11A-11F****: (A)** Spheroid formation in different plate types in Spheroid Maintenance Medium (MM2) and 10% GELTREX®. **(B)** iCell Hepatocytes 2.0 Spheroids form bile canaliculi. (Left: Close-up phase contrast image example of a human iPSC-derived liver spheroid. Right: Confocal image of a ∼40 µm cross-section of a stained spheroid (pHrodo Red AM), with nuclei (Hoechst), and bile canaliculi staining (5(6)-Carboxy-2',7'-dichloro-fluoresceindiacetate; CDCF). The staining for bile canaliculi (white) can be seen throughout the spheroid. **(C)** 3-D liver spheroids display higher levels of urea and CYP3A4 activity than 2-D Cultures. **(D)** Characterization of Cytochrome P450 phase I enzyme activity. Higher activity seen in 3-D versus 2-D cultures. **(E)** Liver spheroid formation from different iPSC donors. **(F)** iPSC-derived hepatocyte spheroids exhibit improved CYP3A induction.

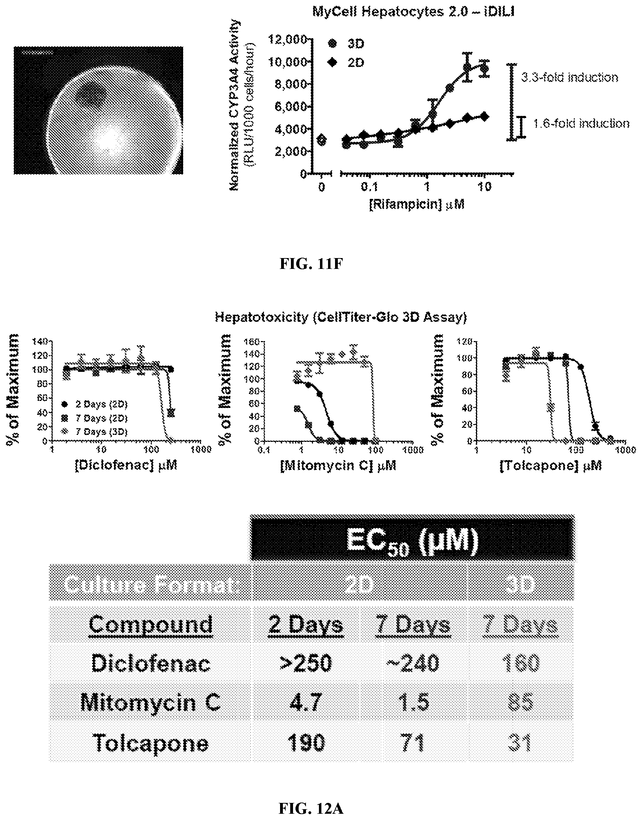

**FIGS. 12A-12D****: (A)** Hepatotoxicity responses for 2-D and 3-D culture formats. **(B)** Modeling for drug-induced liver injury (DILI). **(C)** Hepatotoxicity assay miniaturization. **(D)** Confocal maximum projection images generated during co-culture spheroid formation (left) and transformed into a contour map rendering (right) to help visualize relative positioning of the two cells types within the spheroid. The iCell Hepatocytes (light) and iCell Macrophages (dark) are shown.

### DESCRIPTION OF ILLUSTRATIVE EMBODIMENTS

Limitations with tumor-derived or primary human liver cell models have spurred efforts to develop alternative stem-cell derived human liver cells that recapitulate many of the salient features of primary human liver cells. Previous methods have described how to prepare stem cell-derived human liver cells for use in traditional two-dimensional (2-D) cell cultures; however, these 2-D cultures have limited predictive ability. The present disclosure overcomes several major problems with current technologies by providing methods to produce three-dimensional (3-D) microtissue from pluripotent stem cell (PSC)-derived hepatocytes, where the PSC-derived hepatocytes are supplemented with cell adhesion promoting components and cultured on a non-adhesive surface to produce 3-D microtissue. Notably, the methods of the present disclosure apply to hepatocyte-like cells derived from any type of pluripotent stem cells, including, for example, embryonic stem cells or induced pluripotent stem cells.

In one particular method, cryopreserved induced pluripotent stem (iPS) cell-derived human hepatocytes are used as the source material. First, cells are pre-plated onto traditional 2-D cell culture plates to allow the cells to recover from thaw prior to gentle dissociation and preparation of 3-D microtissue. Alternatively, cells may be thawed directly into formats used to prepare 3-D microtissue cultures.

Next, human microtissue may be prepared by mixing the dissociated hepatocytes with cell-to-cell adhesion-promoting components *(i.e.,* extracellular matrix (ECM) proteins or recombinant protein derivatives), and then dispensing them into cell culture vessels with non-adhesive properties to effectively form microtissue. In one example, PSC-derived hepatocytes are supplemented with a recombinant collagen type I peptide (*e.g*. CELLNEST™) to coat the cells and increase the cell-to-cell adherence.

In particular embodiments, the methods of the present disclosure do not require co-culture with non-parenchymal cells or coating of the cell culture surface such as by extracellular matrix. Additionally, the size of the microtissue can be tuned by modulating the number of cells that are seeded into each cell culture vessel. The microtissue can also be maintained for extended periods of time in culture (e.g., at least 35 days). Further, the 3-D microtissue produced by methods of the present disclosure displays viability and liver-specific functionality as characterized by assessment of the drug-induced cytochrome P450 activity of the microtissue such as in response to treatment by Rifampicin. In particular, the 3-D microtissue has increased cytochrome P450 activity relative to traditional 2-D culture of the PSC-derived hepatocytes.

Thus, in preferred embodiments, the methods of the present disclosure provide 3-D, functional microtissue for a wide range of applications that include model systems for the development of new treatments for a spectrum of liver diseases, the establishment of platforms for predictive toxicology and the creation of *in vitro* models of diseases such as fibrosis, steatosis, and viral infection. In addition, the methods described herein can be used to derive patient-specific microtissue for use in clinical applications of hepatocyte transplantation to restore a degree of liver function to a subject needing such therapy, perhaps due to an acute, chronic, or inherited impairment of liver function.

### I. Definitions

As used herein, "essentially free," in terms of a specified component, is used herein to mean that none of the specified component has been purposefully formulated into a composition and/or is present only as a contaminant or in trace amounts. The total amount of the specified component resulting from any unintended contamination of a composition is therefore well below 0.05%, preferably below 0.01%. Most preferred is a composition in which no amount of the specified component can be detected with standard analytical methods.

As used herein the specification, "a" or "an" may mean one or more. As used herein in the claim(s), when used in conjunction with the word "comprising," the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

The term "cell" is herein used in its broadest sense in the art and refers to a living body that is a structural unit of tissue of a multicellular organism, is surrounded by a membrane structure that isolates it from the outside, has the capability of self-replicating, and has genetic information and a mechanism for expressing it. Cells used herein may be naturally-occurring cells or artificially modified cells (*e.g.,* fusion cells, genetically modified cells, *etc.*).

The term "stem cell" refers herein to a cell that under suitable conditions is capable of differentiating into a diverse range of specialized cell types, while under other suitable conditions is capable of self-renewing and remaining in an essentially undifferentiated pluripotent state. The term "stem cell" also encompasses a pluripotent cell, multipotent cell, precursor cell and progenitor cell. Exemplary human stem cells can be obtained from hematopoietic or mesenchymal stem cells obtained from bone marrow tissue, embryonic stem cells or embryonic germ cells obtained from genital tissue of a fetus. Exemplary pluripotent stem cells can also be produced from somatic cells by reprogramming them to a pluripotent state by the expression of certain transcription factors associated with pluripotency; these cells are called "induced pluripotent stem cells" or "iPSCs".

An "embryonic stem (ES) cell" is an undifferentiated pluripotent stem cell which can be obtained from an embryo in an early stage, such as the inner cell mass at the blastocyst stage, or can be produced by artificial means (*e.g.* nuclear transfer) and can give rise to any differentiated cell type in an embryo or an adult, including germ cells (*e.g.* sperm and eggs).

"Induced pluripotent stem cells (iPSCs)" are cells generated by reprogramming a somatic cell by expressing or inducing expression of a combination of factors (herein referred to as reprogramming factors). iPSCs can be generated using fetal, postnatal, newborn, juvenile, or adult somatic cells. In certain embodiments, factors that can be used to reprogram somatic cells to pluripotent stem cells include, for example, Oct4 (sometimes referred to as Oct 3/4), Sox2, c-Myc, Klf4, Nanog, and Lin28. In some embodiments, somatic cells are reprogrammed by expressing at least two reprogramming factors, at least three reprogramming factors, or four reprogramming factors to reprogram a somatic cell to a pluripotent stem cell.

"Reprogramming" is a process that confers on a cell a measurably increased capacity to form progeny of at least one new cell type, either in culture or *in vivo,* than it would have under the same conditions without reprogramming. More specifically, reprogramming is a process that confers on a somatic cell a pluripotent potential. This means that after sufficient proliferation, a measurable proportion of progeny have phenotypic characteristics of the new cell type if essentially no such progeny could form before reprogramming; otherwise, the proportion having characteristics of the new cell type is measurably more than before reprogramming. Under certain conditions, the proportion of progeny with characteristics of the new cell type may be at least about 0.05%, 0.1%, 0.5%, 1%, 5%, 25% or more in order of increasing preference.

"Pluripotent stem cell" refers to a stem cell that has the potential to differentiate into all cells found in an organism preferably, cells representing any of the three germ layers: endoderm (interior stomach lining, gastrointestinal tract, the lungs), mesoderm (muscle, bone, blood, urogenital), or ectoderm (epidermal tissues and nervous system).

As used herein, the term "somatic cell" refers to any cell other than germ cells, such as an egg, a sperm, or the like, which does not directly transfer its DNA to the next generation. Typically, somatic cells have limited or no pluripotency. Somatic cells used herein may be naturally-occurring or genetically modified.

As used herein the term "engineered" in reference to cells refers to cells that comprise at least one genetic element exogenous to the cell that is integrated into the cell genome. In some aspects, the exogenous genetic element can be integrated at a random location in the cell genome. In other aspects, the genetic element is integrated at a specific site in the genome. For example, the genetic element may be integrated at a specific position to replace an endogenous nucleic acid sequence, such as to provide a change relative to the endogenous sequence (*e.g*., a change in single nucleotide position).

The term "defined" or "fully defined," when used in relation to a medium, an extracellular matrix, or a culture condition, refers to a medium, an extracellular matrix, or a culture condition in which the chemical composition and amounts of approximately all the components are known. For example, a defined medium does not contain undefined factors such as in fetal bovine serum, bovine serum albumin or human serum albumin. Generally, a defined medium comprises a basal media (*e.g*., Dulbecco's Modified Eagle's Medium (DMEM), F12, or Roswell Park Memorial Institute Medium (RPMI) 1640, containing amino acids, vitamins, inorganic salts, buffers, antioxidants and energy sources) which is supplemented with recombinant albumin, chemically defined lipids, and recombinant insulin. An exemplary fully defined medium is Essential 8™ medium.

The term "hepatocyte" as used herein is meant to include hepatocyte-like cells that exhibit some but not all characteristics of mature hepatocytes, as well as mature and fully functional hepatocytes which have all characteristics of hepatocytes as determined by morphology, marker expression, and *in vitro* and *in vivo* functional assays.

The term "drug" or "candidate compound" refers to a molecule including, but not limited to, small molecules, nucleic acids and proteins or combinations thereof that alter or are candidates for altering a phenotype associated with disease.

The term "extracellular matrix protein" refers to a molecule which provides structural and biochemical support to the surrounding cells. The extracellular matrix protein can be recombinant and also refers to fragments or peptides thereof. Examples include collagen and heparin sulfate.

The term "microtissue" refers to a 3-D, spheroidal cell aggregate of human hepatocytes, which is interconnected by intercellular junctions, such as tight junctions. Microtissues may also contain other cell types that are commonly found in the human liver, including, but not limited to, endothelial cells or macrophages. The presence of intercellular junctions can be detected by numerous methods known in the art, *e.g.* by transmission electron microscopy. The microtissues obtained by the method of the present disclosure can have a size which renders them suitable for being used as predictive *in vitro* models of the liver such as for hepatotoxicity. Preferably, the diameter of a microtissue is at least 50 µm, at least 100 µm, at least 150 µm, at least 200 µm, or more. The terms "microtissue", "spheroid", "organoid", and "hepatosphere" are used interchangeably herein.

A "three-dimensional (3-D) culture" refers to an artificially-created environment in which biological cells are permitted to grow or interact with their surroundings in all three dimensions. The 3-D culture can be grown in various cell culture containers such as bioreactors, small capsules in which cells can grow into spheroids, or non-adherent culture plates. In particular aspects, the 3-D culture is scaffold-free. In contrast, a "two-dimensional (2-D)" culture refers to a cell culture such as a monolayer on an adherent surface.

A cell "essentially non-adhesive" surface refers to a surface to which adhesion-dependent cells do not adhere or do not easily adhere. In some aspects, less than 5%, preferably less than 1%, of the cells in the culture adhere to the non-adhesive surface.

As used herein, the term "viscous" applies to a liquid that has a viscosity substantially greater than that of water such as a viscosity that is more than two times the viscosity of water at 25°C. In particular aspects, the PSC-derived hepatocyte cell suspension supplemented with extracellular matrix proteins has a viscosity that is less than two times, such as less than 1.5 times, than that of water at 25°C.

The term "scaffold" refers to a porous material that allows cells seeded thereto to grow on the surface and/or within the pores. For example, the porous material is a common gelling agent, *e.g*., alginate, agar, carrageenan, processed euchema seaweed, locust bean gum, guar gum, tragacanth, acacia gum, xanthan gum, tara gum, gellan, pectin and celluloses (*e.g*. methylcellulose) or an extracellular matrix protein such as collagen.

### II. Production of Microtissue

Embodiments of the present disclosure provide methods of producing 3-D microtissue from pluripotent stem cell (PSC)-derived hepatocytes such as human induced pluripotent stem cell (iPSC)-derived hepatocytes or embryonic stem cell (ESC)-derived hepatocytes.

### A. Pluripotent Stem Cell-Derived Hepatocytes

In certain embodiments of the present disclosure, there are disclosed methods and compositions for producing 3-D microtissue from pluripotent stem cell (PSC)-derived hepatocytes or primary hepatocytes. In some embodiments, the starting population of hepatocytes may be stem cell-derived, including but are not limited to, induced pluripotent stem cells and embryonic stem cells.

### 1. Pluripotent Stem Cells

The starting population of PSC-derived hepatocytes can be derived from human embryonic stem cells (ESCs) and induced pluripotent stem cells (iPSCs). Both ESCs and iPSCs are capable of long-term proliferation *in vitro,* while retaining the potential to differentiate into all cell types of the body, including hepatocytes. The starting populations of human ESC- and iPSC-derived hepatocytes generally do not exhibit the full functional spectrum of human primary adult hepatocytes. Certain aspects of the present disclosure concern a starting population of PSC-derived hepatocytes that could be induced directly from human ESC or iPSCs via expression of a combination of transcription factors for hepatocyte differentiation/function, similar to the generation of iPSCs, bypassing most, if not all, normal developmental stages (US Patent 8,481,317; U.S. Patent Publication No. 20140242595; PCT/US2015/026583).

### a. Embryonic Stem Cells

In certain aspects, the starting population of hepatocytes is derived from embryonic stem cells. ES cells can be derived from the inner cell mass of blastocysts and have a high *in vitro* differentiating capability. ES cells can be isolated by removing the outer trophectoderm layer of a developing embryo, then culturing the inner mass cells on a feeder layer of non-growing cells. The replated cells can continue to proliferate and produce new colonies of ES cells which can be removed, dissociated, replated again and allowed to grow. This process of "subculturing" undifferentiated ES cells can be repeated a number of times to produce cell lines containing undifferentiated ES cells (U.S. Patent Nos. 5,843,780; 6,200,806; 7,029,913). ES cells have the potential to proliferate while maintaining their pluripotency. For example, ES cells are useful in research on cells and on genes which control cell differentiation. The pluripotency of ES cells combined with genetic manipulation and selection can be used for gene analysis studies *in vivo* via the generation of transgenic, chimeric, and knockout mice.

Methods for producing mouse ES cells are well known. In one method, a preimplantation blastocyst from the 129 strain of mice is treated with mouse antiserum to remove the trophoectoderm, and the inner cell mass is cultured on a feeder cell layer of chemically inactivated mouse embryonic fibroblasts in medium containing fetal calf serum. Colonies of undifferentiated ES cells that develop are subcultured on mouse embryonic fibroblast feeder layers in the presence of fetal calf serum to produce populations of ES cells. In some methods, mouse ES cells can be grown in the absence of a feeder layer by adding the cytokine leukemia inhibitory factor (LIF) to serum-containing culture medium (Smith, 2000). In other methods, mouse ES cells can be grown in serum-free medium in the presence of bone morphogenetic protein and LIF (Ying *et al.,* 2003).

Human ES cells can be produced or derived from a zygote or blastocyst-staged mammalian embryo produced by the fusion of a sperm and egg cell, nuclear transfer, pathogenesis, or the reprogramming of chromatin and subsequent incorporation of the reprogrammed chromatin into a plasma membrane to produce an embryonic cell by previously described methods (Thomson and Marshall, 1998; Reubinoff *et al.,* 2000). In one method, human blastocysts are exposed to anti-human serum, and trophectoderm cells are lysed and removed from the inner cell mass which is cultured on a feeder layer of mouse embryonic fibroblasts. Further, clumps of cells derived from the inner cell mass are chemically or mechanically dissociated, replated, and colonies with undifferentiated morphology are selected by micropipette, dissociated, and replated. In some methods, human ES cells can be grown without serum by culturing the ES cells on a feeder layer of fibroblasts in the presence of basic fibroblast growth factor (Amit *et al.,* 2000). In other methods, human ES cells can be grown without a feeder cell layer by culturing the cells on a protein matrix such as MATRIGEL™ or laminin in the presence of "conditioned" medium containing basic fibroblast growth factor (Xu *et al.,* 2001).

ES cells can also be derived from other organisms including rhesus monkey and marmoset by previously described methods (Thomson and Marshall, 1998; Thomson *et al.,* 1995; Thomson and Odorico, 2000; U.S. Patent No. 5,843,780), as well as from established mouse and human cell lines. For example, established human ES cell lines include MAOI, MA09, ACT-4, HI, H7, H9, H13, H14 and ACT30. cited for reference only. As a further example, mouse ES cell lines that have been established include the CGR8 cell line established from the inner cell mass of the mouse strain 129 embryos, and cultures of CGR8 cells can be grown in the presence of LIF without feeder layers.

ES stem cells can be detected by protein markers including transcription factor Oct4, alkaline phosphatase (AP), stage-specific embryonic antigen SSEA-1, stage-specific embryonic antigen SSEA-3, stage-specific embryonic antigen SSEA-4, transcription factor NANOG, tumor rejection antigen 1-60 (TRA-1-60), tumor rejection antigen 1-81 (TRA-1-81), SOX2, or REX1.

### b. Induced Pluripotent Stem Cells

In other aspects, the starting population of hepatocytes is derived from induced pluripotent stem cells, commonly abbreviated iPS cells or iPSCs. The induction of pluripotency was originally achieved in 2006 using mouse cells (Yamanaka *et al.* 2006) and in 2007 using human cells (Yu *et al.* 2007; Takahashi *et al.* 2007) by reprogramming of somatic cells via the introduction of transcription factors that are linked to pluripotency. The use of iPSCs circumvents most of the ethical and practical problems associated with large-scale clinical use of ES cells, and patients with iPSC-derived autologous transplants may not require lifelong immunosuppressive treatments to prevent graft rejection.

With the exception of certain cell types (such as germ cells and enucleated erythrocytes), any cell can be used as a starting point for iPSCs. For example, cell types could be keratinocytes, fibroblasts, hematopoietic cells, mesenchymal cells, liver cells, or stomach cells. T cells may also be used as a source of somatic cells for reprogramming (U.S. Patent Publication No. 20140315304; U.S. Patent No. 8,741,648). There is no limitation on the degree of cell differentiation or the age of an animal from which cells are collected; even undifferentiated progenitor cells (including somatic stem cells) and finally differentiated mature cells can be used as sources of somatic cells in the methods disclosed herein. In one embodiment, the somatic cell is itself a blood cell such as a human CD34+ hematopoietic progenitor cell or a skin cell such as a human fibroblast. The somatic cell can be an adult or a fetal somatic cell. iPSCs can be grown under conditions that are known to differentiate human ES cells into specific cell types, and express human ES cell markers including: SSEA-1, SSEA-3, SSEA-4, TRA-1-60, and TRA-1-81.

Somatic cells can be reprogrammed to produce induced pluripotent stem cells (iPSCs) using methods known to one of skill in the art. One of skill in the art can readily produce induced pluripotent stem cells, see for example, Published U.S. Patent Application No. 20090246875, Published U.S. Patent Application No. 2010/0210014; Published U.S. Patent Application No. 20120276636; U.S. Patent No. 8,058,065; U.S. Patent No. 8,129,187; PCT Publication NO. WO 2007/069666 A1, and U.S. Patent No. 8,268,620, which are incorporated herein by reference. Generally, nuclear reprogramming factors are used to produce pluripotent stem cells from a somatic cell. In some embodiments, at least three, or at least four, of Klf4, c-Myc, Oct3/4, Sox2, Nanog, and Lin28 are utilized. In other embodiments, Oct3/4, Sox2 and at least one of Nanog and Lin28 are utilized. In other embodiments, Oct3/4, Sox2 and at least one of c-Myc and Klf4 are utilized.

Mouse and human cDNA sequences of these nuclear reprogramming substances are available with reference to the NCBI accession numbers mentioned in US Patent No. 8,183,038 and WO 2007/069666. Methods for introducing one or more reprogramming substances, or nucleic acids encoding these reprogramming substances, are known in the art, and disclosed for example, in published U.S. Patent Nos. 8,071,369, 8,268,620, 8,691,574, 8,741,648, 8,546,140, 8,900,871 and 9,175,268.

Once derived, iPSCs can be cultured in a medium sufficient to maintain pluripotency. The iPSCs may be used with various media and techniques developed to culture pluripotent stem cells, more specifically, embryonic stem cells, as described in U.S. Patent No. 7,442,548 and U.S. Patent Pub. No. 2003/0211603. In the case of mouse cells, the culture is carried out with the addition of Leukemia Inhibitory Factor (LIF) as a differentiation suppression factor to an ordinary medium. In the case of human cells, it is desirable that basic fibroblast growth factor (bFGF) be added in place of LIF. Other methods for the culture and maintenance of iPSCs, as would be known to one of skill in the art, may be used with the present disclosure.

In certain embodiments, undefined conditions may be used; for example, pluripotent cells may be cultured on fibroblast feeder cells or a medium that has been exposed to fibroblast feeder cells in order to maintain the stem cells in an undifferentiated state. In some embodiments, the cell is cultured in the co-presence of mouse embryonic fibroblasts treated with radiation or an antibiotic to terminate the cell division, as feeder cells. Alternately, pluripotent cells may be cultured and maintained in an essentially undifferentiated state using a defined, feeder-independent culture system, such as a TESR™ medium (Ludwig *et al.,* 2006a; Ludwig *et al.,* 2006b) or E8™ /Essential 8™ medium (Chen *et al.,* 2011).

Plasmids have been designed with a number of goals in mind, such as achieving regulated high copy number and avoiding potential causes of plasmid instability in bacteria, and providing means for plasmid selection that are compatible with use in mammalian cells, including human cells. Particular attention has been paid to the dual requirements of plasmids for use in human cells. First, they are suitable for maintenance and fermentation in E. coli, so that large amounts of DNA can be produced and purified. Second, they are safe and suitable for use in human patients and animals. The first requirement calls for high copy number plasmids that can be selected for and stably maintained relatively easily during bacterial fermentation. The second requirement calls for attention to elements such as selectable markers and other coding sequences. In some embodiments plasmids that encode a marker are composed of: (1) a high copy number replication origin, (2) a selectable marker, such as, but not limited to, the neo gene for antibiotic selection with kanamycin, (3) transcription termination sequences, including the tyrosinase enhancer and (4) a multicloning site for incorporation of various nucleic acid cassettes; and (5) a nucleic acid sequence encoding a marker operably linked to the tyrosinase promoter. There are numerous plasmid vectors that are known in the art for inducing a nucleic acid encoding a protein. These include, but are not limited to, the vectors disclosed in U.S. Patent No. 6,103,470; U.S. Patent No. 7,598,364; U.S. Patent No. 7,989,425; and U.S. Patent No. 6,416,998.

An episomal gene delivery system can be a plasmid, an Epstein-Barr virus (EBV)-based episomal vector (U.S. Patent 8,546,140), a yeast-based vector, an adenovirus-based vector, a simian virus 40 (SV40)-based episomal vector, a bovine papilloma virus (BPV)-based vector, or a lentiviral vector. A viral gene delivery system can be an RNA-based or DNA-based viral vector (PCT/JP2009/062911, PCT/JP2011/069588).

### c. Embryonic Stem Cells Derived by Somatic Cell Nuclear Transfer

Pluripotent stem cells for deriving the starting population of hepatocytes could also be prepared by means of somatic cell nuclear transfer, in which a non human donor nucleus is transferred into a spindle-free non human oocyte. Stem cells produced by nuclear transfer are genetically identical to the donor nuclei. In one method, donor fibroblast nuclei from skin fibroblasts of a rhesus macaque are introduced into the cytoplasm of spindle-free, mature metaphase II rhesus macaque ooctyes by electrofusion (Byrne *et al.,* 2007). The fused oocytes are activated by exposure to ionomycin, then incubated until the blastocyst stage. The inner cell mass of selected blastocysts are then cultured to produce embryonic stem cell lines. The embryonic stem cell lines show normal ES cell morphology, express various ES cell markers, and differentiate into multiple cell types both *in vitro* and *in vivo.*

### 2. Programming Factors for PSC-Derived Hepatocytes

Certain aspects of the present disclosure concern a starting population of iPSC-derived hepatocytes produced by hepatocyte programming factors for hepatocyte forward programming. The hepatocytes could be produced directly from PSCs by increasing the level of hepatocyte programming factors in the cells (US Patent 8,481,317; U.S. Patent Publication No. 20140242595; PCT/US2015/026583). The numerous functions of hepatocytes could be controlled at the transcriptional level by the concerted actions of a limited number of hepatocyte-enriched transcription factors. Any transcription factors with a role in hepatocyte differentiation or function may be used to produce the starting population of PSC-derived hepatocytes described herein, like hepatocyte-enriched transcription factors, particularly the genes thereof listed in Table 1.

For example, by effecting expression of a combination of transcription factors in Table 1, forward programming into hepatocytes from pluripotent stem cells may be used to obtain PSC-derived hepatocytes. For example, the PSC-derived hepatocytes can be derived by a combination of the following transcription factors: FOXA2, HHEX, HNF1A, GATA4, NR1I3, MAFB, and TBX3.

**Table 1. A list of candidate genes for direct programming of human ESC or iPSCs to hepatocytes.**

| # | **Symbol** | **Entrez Gene ID** | **Accession** | **Name** |
|---|---|---|---|---|
| 1 | FOXA1 | 3169 | NM_004496 | forkhead box A1 |
| 2 | FOXA2 | 3170 | NM_021784 | forkhead box A2 isoform 1 |
| | | | NM_153675 | forkhead box A2 isoform 2 |
| 3 | FOXA3 | 3171 | NM_004497 | forkhead box A3 |
| 4 | GATA4 | 2626 | NM_002052 | GATA binding protein 4 |
| 5 | HHEX | 3087 | NM_002729 | hematopoietically expressed homeobox |
| 6 | TBX3 | 6926 | NM_005996 | T-box 3 isoform 1 |
| | | | NM_016569 | T-box 3 isoform 2 |
| 7 | HNF1A | 6927 | NM_000545 | HNF1 homeobox A |
| 8 | HNF4A | 3172 | NM_000457 | hepatocyte nuclear factor 4, alpha |
| 9 | MAFB | 9935 | NM_005461 | v-maf musculoaponeurotic fibrosarcoma oncogene homolog B (avian) |
| 10 | ABLIM3 | 22885 | NM_014945 | actin binding LIM protein family, member 3 |
| 11 | AHR | 196 | NM_001621 | aryl hydrocarbon receptor |
| 12 | AR | 367 | NM_000044 | androgen receptor |
| 13 | ATF5 | 22809 | NM_012068 | activating transcription factor 5 |
| 14 | ATOH8 | 84913 | NM_032827 | atonal homolog 8 (Drosophila) |
| 15 | ESR1 | 2099 | NM_000125 | estrogen receptor 1 |
| 16 | NF1A | 4774 | NM_001134 673 | nuclear factor I/A |
| 17 | NF1B | 4781 | NM_005596 | nuclear factor I/B |
| 18 | NR0B2 | 8431 | NM_021969 | nuclear receptor subfamily 0, group B, member 2 |
| 19 | NR1H4 | 9971 | NM_005123 | nuclear receptor subfamily 1, group H, member 4 |
| 20 | NR1I2 | 8856 | NM_003889 | nuclear receptor subfamily 1, group I, member 2, isoform 1 |
| | | | NM_022002 | nuclear receptor subfamily 1, group I, member 2, isoform 2 |
| 21 | NR1I3 | 9970 | NM_001077 482 | nuclear receptor subfamily 1, group I, member 3, transcript variant 1 |
| 22 | NR3C2 | 4306 | NM_000901 | nuclear receptor subfamily 3, group C, member 2 |
| 23 | NR5A2-2 | 2494 | NM_003822 | nuclear receptor subfamily 5, group A, member 2 |
| 24 | PPARA | 5465 | NM_005036 | PPARA peroxisome proliferator-activated receptor alpha |
| 25 | PROX1 | 5629 | NM_002763 | prospero homeobox 1 |
| 26 | RORC | 6097 | NM_005060 | RAR-related orphan receptor C |
| 27 | SCML1 | 6322 | NM_001037 540 | sex comb on midleg-like 1 (Drosophila) isoform a |
| | | | NM_006746 | sex comb on midleg-like 1 (Drosophila) isoform b |
| | | | NM_001037 535 | sex comb on midleg-like 1 (Drosophila) isoform c |
| 28 | THRB | 7068 | NM_000461 | thyroid hormone receptor, beta (erythroblastic leukemia viral (v-erb-a) oncogene homolog 2, avian) |
| 29 | ZIC1 | 7545 | NM_003412 | Zic family member 1 (odd-paired homolog, Drosophila) |

### B. Methods of Producing Microtissue

### 1. Cell Suspension of PSC-Derived Hepatocytes

In certain embodiments, the present disclosure provides methods of producing functional, 3-D microtissue from a starting cell suspension of PSC-derived hepatocytes such as iCell® Hepatocytes. A starting cell suspension of PSC-derived hepatocytes can be obtained from a 2-D culture of PSC-derived hepatocytes or directly from cryopreserved PSC-derived hepatocytes. In some aspects, the cryopreserved PSC-derived hepatocytes are thawed to obtain the cell suspension and directly cultured in a 3-D culture. Alternatively, the cryopreserved PSC-derived hepatocytes can be pre-plated in a traditional 2-D culture to allow the cells to recover. Methods of 2-D culture are known in the art. In one method, the thawed hepatocytes can be resuspended in medium such as Plating Medium (Table 2) to prepare a cell suspension. In one particular method, the thawed hepatocytes are resuspended in Plating Medium 2 (Table 6, comprising 98% DMEM/F-12 Medium, IX B27 Supplement, 0.1 µM dexamethasone, 25 µg/mL Gentamicin, and 20 ng/mL Oncostatin M). Cryopreserved cells may be thawed, e.g., at a temperature of about 25° C to about 40° C, and typically at a temperature of about 37° C.

In certain aspects, a starting cell suspension for the present methods may comprise at least or about 10⁴, 10⁵, 10⁶, 10⁷, 10⁸, 10⁹, 10¹⁰, 10¹¹, 10¹², 10¹³ cells or any range derivable therein. The starting cell population may have a seeding density of at least or about 10, 10¹, 10², 10³, 10⁴, 10⁵, 10⁶, 10⁷, 10⁸ cells/mL, or any range derivable therein.

The thawed PSC-derived hepatocytes can be seeded into a cell culture vessel such as with a cell-adhesive bottom (e.g., coated with a matrix). The cellular adhesive culture vessel can be coated with any substrate for cell adhesion such as extracellular matrix (ECM) to improve the adhesiveness of the vessel surface to the cells. The substrate used for cell adhesion can be any material intended to attach stem cells or feeder cells (if used). Non-limiting substrates for cell adhesion include collagen, gelatin, poly-L-lysine, poly-D-lysine, poly-L-ornithine, laminin, vitronectin, and fibronectin and mixtures thereof, for example, protein mixtures from Engelbreth-Holm-Swarm mouse sarcoma cells (such as Matrigel™ or GELTREX®) and lysed cell membrane preparations (Klimanskaya *et al.,* 2005). The PSC-derived hepatocytes can be cultured for a period of time sufficient to recover from cryopreservation such as about 1 day to about 15 days, such as about 2, 3, 4, 5, 6, 7, 8, 9, or 10 days.

After the PSC-derived hepatocytes have recovered from cryopreservation, the cells are preferably detached from the cell surface to obtain a cell suspension, such as by the addition of a cell detachment reagent. The colony is split into aggregated cells or even single cells by any method suitable for dissociation, which cells are then placed into new culture containers for passaging. Cell passaging or splitting is a technique that enables cells to survive and grow under cultured conditions for extended periods of time. Cells typically would be passaged when they are about 70%-100% confluent.

Any cell detachment reagent known in the art can be used to dissociate the PSC-derived hepatocytes. For example, the cell detachment reagent is ACCUTASE®, TRYPLE™, ACCUMAX™, or Trypsin. In particular, the cell detachment reagent is ACCUTASE®. In certain embodiments, pluripotent stem cells may be dissociated into single individual cells, or a combination of single individual cells and small cell clusters comprising 2, 3, 4, 5, 6, 7, 8, 9, 10 cells or more. In particular aspects, the dissociation does not disrupt clusters of cells.

### 2. Supplementation by Cell-Adhesion Promoting Components

Once a cell suspension of PSC-derived is obtained, the cell suspension is supplemented with cell-adhesion promoting components such as extracellular matrix proteins or peptides thereof. In certain aspects, the supplementation is at a low concentration to prevent the cell suspension from gelling or becoming viscous. The low percentage of extracellular matrix proteins (ECM) can be from about 0.1 percent to about 30 percent of the cell suspension volume, such as about 5 percent to about 20 percent of the cell suspension. In some embodiments, the total ECM protein concentration in the cell suspension can range from about 1 ng/mL to about 0.1 mg/mL, such as about 0.005 mg/mL to about 0.05 mg/mL. In particular embodiments, the total protein concentration of matrix supplementation is about 15 ug/mL for GELTREX® and about 200 ug/mL for CELLNEST™ (*e.g*., US20120329157).

For example, extracellular matrix components include one or more of the following proteins: fibronectin, laminin, vitronectin, tenascin, entactin, thrombospondin, elastin, gelatin, collagen, fibrillin, merosin, anchorin, chondronectin, link protein, bone sialoprotein, osteocalcin, osteopontin, epinectin, hyaluronectin, undulin, epiligrin, and kalinin. In exemplary methods, the cell suspension is supplemented with a mixture laminin, collagen type IV, entactin, and heparin sulfate proteoglycan (*e.g.*, GELTREX®).

In other methods, the cell suspension is supplemented with recombinant peptide of collagen type I such as CELLNEST™ as described in U.S. Patent Application No. US20130329157 (*e.g*., paragraphs [0071-0088]) and International Patent Application No. WO2008103041 as the recombinant gelatin CBE3 with a molecular weight of 51.6 kD. CELLNEST™ (Fujifilm) is a non-animal recombinant peptide (RCP) based on human collagen type I (α I chain) that is enriched with arginine-glycine-aspartate (RGD) sequences to enhance cell adhesion.

The extracellular matrix (ECM) proteins may be of natural origin and purified from human or animal tissues or, alternatively, the ECM proteins may be genetically engineered recombinant proteins or synthetic in nature. The ECM proteins may be a whole protein or in the form of peptide fragments, native or engineered. In some embodiments, the cell suspension is supplemented by synthetically generated peptide fragments of collagen or recombinant collagen. In some embodiments, the ECM components are xeno-free.

The extracellular matrix supplementation comprises at least one extracellular matrix protein or fragment thereof. Extracellular matrix proteins for use as in the supplementation include, but are not limited to, collagen I, collagen III, collagen IV, fibronectin, laminin, vitronectin, gelatin, or combinations thereof. In some embodiments, the ECM supplementation is GELTREX® matrix (Life Technologies Corporation). GELTREX® matrix is composed in part of collagen IV, entactin, and laminin. The ECM supplementation can be made from purified individual ECM components, from basement membrane extracted from tumor cells, or from ECM isolated from tissue, such as, for example, extracted ECM from liver. Extracellular matrix proteins, ECM components, and ECM matrices are commercially available.

### 3. Non-Adhesive Cell Culture Surface

The cell suspension of PSC-derived hepatocytes supplemented with cell-adhesion promoting components can then be seeded in an appropriate culture vessel with a cell non-adhesive surface, such as an ultra-low attachment tissue culture plate, such as a flask, 6-well, 24-well, 96-well, or 384-well plate. For example, the ultra-low attachment (ULA) plate is a CORNING® round-bottom ULA plate or an InSphero GravityTRAP™ ULA plate. Ultra-low attachment plates are readily known and commercially available. For example, the GravityTRAP™ ULA plate is a scaffold free, ultra-low attachment plate that is non-adhesively coated by hydrophilic, neutrally charged coating. Any cell non-adhesive surface may be employed as long as it is a surface to which adhesion-dependent cells do not adhere or do not easily adhere. Examples of such surfaces may include a bottom of a culture container made of such materials as polystyrene, polypropylene, fluororesin, polytetrafluoroethylene (PTFE), polycarbonate, polyester, a positively charged bottom of a culture container, and the like.

A culture vessel used for culturing the cell(s) can include, but is particularly not limited to: flask, flask for tissue culture, dish, petri dish, dish for tissue culture, multi dish, micro plate, micro-well plate, multi plate, multi-well plate, micro slide, chamber slide, tube, tray, CELLSTACK® Chambers, culture bag, and roller bottle, as long as it is capable of culturing the stem cells therein. The cells may be cultured in a volume of at least or about 0.2, 0.5, 1, 2, 5, 10, 20, 30, 40, 50 mL, 100 mL, 150 mL, 200 mL, 250 mL, 300 mL, 350 mL, 400 mL, 450 mL, 500 mL, 550 mL, 600 ml, 800 mL, 1000 mL, 1500 mL, or any range derivable therein, depending on the needs of the culture. In a certain embodiment, the culture vessel may be a bioreactor, which may refer to any device or system *ex vivo* that supports a biologically active environment such that cells can be propagated. The bioreactor may have a volume of at least or about 2, 4, 5, 6, 8, 10, 15, 20, 25, 50, 75, 100, 150, 200, 500 liters, 1, 2, 4, 6, 8, 10, 15 cubic meters, or any range derivable therein.

In certain aspects, the size of the microtissue will be determined by the number of starting cells. The number of cells used generally ranges from about 1000 to about 1 million. In certain aspects, the cell suspension can be seeded at a cell density of about 100 to about 100,000 cells per well, such as about 2,000 to about 20,000 cells per well. For example, seeding 2,500 cells per well can produce a microtissue with a diameter of about 250 µm and seeding 15,000 cells per well can produce a microtissue of about 600 µm in diameter. Generally, the cells are plated in 3-D formats at a cell density of about 1,000 to about 1,000,000 cells/cm², such as of about 5,000 to about 300,000 cells/cm². In particular embodiments, for pre-plating of the cells in 2-D culture, such as a 6-well 2-D culture plate (surface area of about 10 cm² per well), the cells may be seeded at a cell density of about 500,000 to about 5,000,000 cells per well.

Generally, the PSC-derived hepatocytes are cultured on the non-adhesive surface for a length of time sufficient to produce the 3-D microtissue. In exemplary methods, the hepatocytes are cultured for about 24 hours to about 72 hours, such as about 48 hours, to obtain functional microtissue. The microtissue can be maintained by replacing the medium with fresh medium such as every other day for about 2 days to about 4 weeks.

In certain aspects, non-static culture could be used for producing microtissue. The non-static culture can be any culture with cells kept at a controlled moving speed, by using, for example, shaking, rotating, or stirring platforms or culture vessels, particularly large-volume rotating bioreactors. The agitation may improve circulation of nutrients and cell waste products and also be used to control cell aggregation by providing a more uniform environment. For example, rotary speed may be set to at least or at most about 25, 30, 35, 40, 45, 50, 75, 100 rpm, or any range derivable therein. The incubation period in the non-static culture may be at least or about 4 hours, 8 hours, 16 hours, or 1, 2, 3, 4, 5, 6 days, or 1, 2, 3, 4, 5, 6, 7 weeks, or any range derivable therein.

The hepatocytes can be cultured with the nutrients necessary to support their growth. Generally, the cells are cultured in growth media including a carbon source, a nitrogen source and a buffer to maintain pH. The medium can also contain fatty acids or lipids, amino acids (such as non-essential amino acids), vitamin(s), growth factors, cytokines, antioxidant substances, pyruvic acid, buffering agents, and inorganic salts. An exemplary growth medium contains a minimal essential media, such as Dulbecco's Modified Eagle's medium (DMEM) or ESSENTIAL 8™ (E8™) medium, supplemented with various nutrients, such as non-essential amino acids and vitamins, to enhance stem cell growth. Examples of minimal essential media include, but are not limited to, Minimal Essential Medium Eagle (MEM) Alpha medium, Dulbecco's modified Eagle medium (DMEM), RPMI-1640 medium, 199 medium, and F12 medium. Additionally, the minimal essential media may be supplemented with additives such as horse, calf or fetal bovine serum. Alternatively, the medium can be serum free. In other cases, the growth media may contain "knockout serum replacement," referred to herein as a serum-free formulation optimized to grow and maintain undifferentiated cells, such as stem cell, in culture. KNOCKOUT™ serum replacement is disclosed, for example, in U.S. Patent Application No. 2002/076747. Preferably, the PSC-derived hepatocytes are cultured in a fully defined and feeder free media. In exemplary methods, the PSC-derived hepatocytes are cultured in Plating Medium (Table 2) and maintained in Maintenance Medium (Table 3). In one particular method, the thawed hepatocytes are cultured and recovered from thaw for several days in Plating Medium 2 (Table 6, comprising 98% DMEM/F-12 Medium, IX B27 Supplement, 0.1 µM dexamethasone, 25 µg/mL Gentamicin, and 20 ng/mL Oncostatin M) prior to forming and maintaining spheroids in Maintenance Medium 2 (Table 6, same formulation as Plating Medium 2 only without Oncostatin M).

The medium may contain or may not contain any alternatives to serum. The alternatives to serum can include materials which appropriately contain albumin (such as lipid-rich albumin, albumin substitutes such as recombinant albumin, plant starch, dextrans and protein hydrolysates), transferrin (or other iron transporters), fatty acids, insulin, collagen precursors, trace elements, 2-mercaptoethanol, 3'-thiolglycerol, or equivalents thereto. The alternatives to serum can be prepared by the method disclosed in International Publication No. WO 98/30679, for example. Alternatively, any commercially available materials can be used for more convenience. The commercially available materials include KNOCKOUT™ Serum Replacement (KSR), Chemically-defined Lipid concentrated (Gibco), and GLUTAMAX™ (Gibco).

Other culturing conditions can be appropriately defined. For example, the culturing temperature can be about 30 to 40°C, for example, at least or about 31, 32, 33, 34, 35, 36, 37, 38, 39°C but particularly not limited to them. In one embodiment, the cells are cultured at 37°C. The CO₂ concentration can be about 1 to 10%, for example, about 2 to 5%, or any range derivable therein. The oxygen tension can be at least, up to, or about 1, 2, 3, 4, 5, 6, 7, 8, 9, 10, 20%, or any range derivable therein.

### C. Characterization of Microtissue

The viability and/or functionality of the 3-D microtissue produced by the methods of the present disclosure can be assessed according to a number of phenotypic and/or functional criteria. The criteria include but are not limited to receptivity to viral infection such as HBV infection, the detection or quantitation of expressed cell markers, enzymatic activity, and the characterization of morphological features and intercellular signaling.

In one method, the microtissue is characterized by exhibiting liver-specific markers similar to primary human hepatocytes. Such expressed markers include, but are not limited to, albumin, key drug metabolizing enzymes (*e.g.*, cytochrome P450 enzymes such as CYP3A4), signal transduction pathway components (*e.g.*, nuclear receptors), and drug transporters *(e.g.,* MRP2). See, for example, (Gripon *et al.,* 2002; Parent *et al.,* 2004).

Accordingly, functional microtissue responds to prototypical cytochrome P450 inducers such as omeprazole (OMP), phenobarbital (PB), and rifampicin (RIF). The liver microtissue is also tolerant of long culture periods *(e.g.,* >7 days, >14 days, >21 days) and so are well-suited for *in vitro* determination of acute and chronic toxicity resulting, for example, from intrinsic and/or metabolism-based mechanisms.

### III. Use of Microtissue

Certain aspects provide a method to produce functional, 3-D microtissue which can be used for a number of important research, development, and commercial purposes. The microtissue provided by methods and compositions of certain aspects of the present disclosure can be used in a variety of applications. These include, but are not limited to, transplantation or implantation of the microtissue *in vivo;* screening anti-virals, cytotoxic compounds, carcinogens, mutagens, growth/regulatory factors, pharmaceutical compounds, *etc., in vitro;* elucidating the mechanism of liver diseases and infections; studying the mechanism by which drugs and/or growth factors operate; diagnosing and monitoring cancer in a patient; gene therapy; and the production of biologically active products, to name but a few.

### A. Test Compound Screening

Microtissue of this present disclosure can be used to screen for factors (such as solvents, small molecule drugs, peptides, and polynucleotides) or environmental conditions (such as culture conditions or manipulation) that affect the characteristics of the microtissue provided herein.

Particular screening applications of the present disclosure relate to the testing of pharmaceutical compounds in drug research. The reader is referred generally to the standard textbook In vitro Methods in Pharmaceutical Research, Academic Press, 1997). In certain aspects of the present disclosure, agent-treated hepatocytes play the role of test cells for standard drug screening and toxicity assays, as have been previously performed on hepatocyte cell lines or primary hepatocytes in short-term culture. Assessment of the activity of candidate pharmaceutical compounds generally involves combining the microtissue provided in certain aspects of the present disclosure with the candidate compound, determining any change in the morphology, marker phenotype, or metabolic activity of the cells that is attributable to the compound (compared with untreated cells or cells treated with an inert compound), and then correlating the effect of the compound with the observed change. The screening may be done either because the compound is designed to have a pharmacological effect on liver cells, or because a compound designed to have effects elsewhere may have unintended hepatic side effects. Two or more drugs can be tested in combination (by combining with the cells either simultaneously or sequentially), to detect possible drug-drug interaction effects.

In some applications, compounds are screened initially for potential hepatotoxicity (Castell *et al.,* 1997). Cytotoxicity can be determined in the first instance by the effect on cell viability, survival, morphology, and leakage of enzymes into the culture medium. More detailed analysis is conducted to determine whether compounds affect cell function (such as gluconeogenesis, ureagenesis, and plasma protein synthesis) without causing toxicity. Lactate dehydrogenase (LDH) is a good marker because the hepatic isoenzyme (type V) is stable in culture conditions, allowing reproducible measurements in culture supernatants after 12-24 h incubation. Leakage of enzymes such as mitochondrial glutamate oxaloacetate transaminase and glutamate pyruvate transaminase can also be used. Gomez-Lechon *et al.* (1996) describes a microassay for measuring glycogen, which can be used to measure the effect of pharmaceutical compounds on hepatocyte gluconeogenesis.

Other current methods to evaluate hepatotoxicity include determination of the synthesis and secretion of albumin, cholesterol, and lipoproteins; transport of conjugated bile acids and bilirubin; ureagenesis; cytochrome P450 levels and activities; glutathione levels; release of α-glutathione s-transferase; ATP, ADP, and AMP metabolism; intracellular K+ and Ca2+ concentrations; the release of nuclear matrix proteins or oligonucleosomes; and induction of apoptosis (indicated by cell rounding, condensation of chromatin, and nuclear fragmentation). DNA synthesis can be measured as [³H]-thymidine or BrdU incorporation. Effects of a drug on DNA synthesis or structure can be determined by measuring DNA synthesis or repair. [³H]-thymidine or BrdU incorporation, especially at unscheduled times in the cell cycle, or above the level required for cell replication, is consistent with a drug effect. Unwanted effects can also include unusual rates of sister chromatid exchange, determined by metaphase spread. The reader is referred to Vickers (1997) for further elaboration.

### B. Liver Therapy and Transplantation

The present disclosure also provides for the use of microtissue provided herein to restore a degree of liver function to a subject needing such therapy, perhaps due to an acute, chronic, or inherited impairment of liver function.

To determine the suitability of microtissue provided herein for therapeutic applications, the microtissue can first be tested in a suitable animal model. At one level, microtissues are assessed for their ability to survive and maintain their phenotype *in vivo.* Microtissues provided herein are administered to immunodeficient animals (such as SCID mice, or animals rendered immunodeficient chemically or by irradiation) at a site amenable for further observation, such as under the kidney capsule, into the spleen, or into a liver lobule. Tissues are harvested after a period of a few days to several weeks or more, and assessed as to whether starting cell types such as pluripotent stem cells are still present. This can be performed by providing the administered cells with a detectable label (such as green fluorescent protein, or β-galactosidase); or by measuring a constitutive marker specific for the administered cells. Where microtissues provided herein are being tested in a rodent model, the presence and phenotype of the administered cells can be assessed by immunohistochemistry or ELISA using human-specific antibody, or by RT-PCR analysis using primers and hybridization conditions that cause amplification to be specific for human polynucleotide sequences. Suitable markers for assessing gene expression at the mRNA or protein level are provided in elsewhere in this disclosure. General descriptions for determining the fate of hepatocyte-like cells in animal models is provided in Grompe *et al.* (1999); Peeters *et al.* (1997); and Ohashi *et al.* (2000).

At another level, microtissue provided herein is assessed for its ability to restore liver function in an animal lacking full liver function. Braun *et al.* (2000) outline a model for toxin-induced liver disease in mice transgenic for the HSV-tk gene. Rhim *et al.* (1995) and Lieber *et al.* (1995) outline models for liver disease by expression of urokinase. Mignon *et al.* (1998) outline liver disease induced by antibody to the cell-surface marker Fas. Overturf *et al.* (1998) have developed a model for Hereditary Tyrosinemia Type I in mice by targeted disruption of the Fah gene. The animals can be rescued from the deficiency by providing a supply of 2-(2-nitro-4-fluoro-methyl-benzyol)-1,3-cyclohexanedione (NTBC), but they develop liver disease when NTBC is withdrawn. Acute liver disease can be modeled by 90% hepatectomy (Kobayashi *et al.,* 2000). Acute liver disease can also be modeled by treating animals with a hepatotoxin such as galactosamine, CC14, or thioacetamide.

Chronic liver diseases, such as cirrhosis, can be modeled by treating animals with a sub-lethal dose of a hepatotoxin long enough to induce fibrosis (Rudolph *et al.,* 2000). Assessing the ability of microtissue provided herein to reconstitute liver function involves administering the cells to such animals, and then determining survival over a 1 to 8 week period or more, while monitoring the animals for progress of the condition. Effects on hepatic function can be determined by evaluating markers expressed in liver tissue, cytochrome P450 activity, and blood indicators, such as alkaline phosphatase activity, bilirubin conjugation, and prothrombin time), and survival of the host. Any improvement in survival, disease progression, or maintenance of hepatic function according to any of these criteria relates to effectiveness of the therapy, and can lead to further optimization.

Microtissues provided in certain aspects of the present disclosure that demonstrate desirable functional characteristics according to their profile of metabolic enzymes, or efficacy in animal models, may also be suitable for direct administration to human subjects with impaired liver function. For purposes of hemostasis, the microtissue can be administered at any site that has adequate access to the circulation, typically within the abdominal cavity. For some metabolic and detoxification functions, it is advantageous for the microtissue to have access to the biliary tract. Accordingly, the microtissues are administered near the liver (*e.g.,* in the treatment of chronic liver disease) or the spleen (*e.g.,* in the treatment of fulminant hepatic failure). In one method, the microtissue are administered into the hepatic circulation either through the hepatic artery, or through the portal vein, by infusion through an in-dwelling catheter. A catheter in the portal vein can be manipulated so that the microtissues flow principally into the spleen, or the liver, or a combination of both. In another method, the cells are administered by placing a bolus in a cavity near the target organ, typically in an excipient or matrix that will keep the bolus in place. In another method, the cells are injected directly into a lobe of the liver or the spleen.

The microtissue provided in certain aspects of the present disclosure can be used for therapy of any subject in need of having hepatic function restored or supplemented. Human conditions that may be appropriate for such therapy include fulminant hepatic failure due to any cause, viral hepatitis, drug-induced liver injury, cirrhosis, inherited hepatic insufficiency (such as Wilson's disease, Gilbert's syndrome, or α1-antitrypsin deficiency), hepatobiliary carcinoma, autoimmune liver disease (such as autoimmune chronic hepatitis or primary biliary cirrhosis), and any other condition that results in impaired hepatic function. For human therapy, the dose is generally between about 10⁹ and 10¹² cells, and typically between about 5×10⁹ and 5×10¹⁰ cells, making adjustments for the body weight of the subject, nature and severity of the affliction, and the replicative capacity of the administered cells. The ultimate responsibility for determining the mode of treatment and the appropriate dose lies with the managing clinician.

### C. Use in a Liver Assist Device

Certain aspects of the present disclosure include microtissues provided herein that are encapsulated or part of a bioartificial liver device. Various forms of encapsulation are described in *Cell Encapsulation Technology and Therapeutics,* 1999. Hepatocytes provided in certain aspects of the present disclosure can be encapsulated according to such methods for use either *in vitro* or *in vivo.*

Bioartificial organs for clinical use are designed to support an individual with impaired liver function-either as a part of long-term therapy, or to bridge the time between a fulminant hepatic failure and hepatic reconstitution or liver transplant. Bioartificial liver devices are reviewed by Macdonald *et al.* (1999) and exemplified in U.S. Pat. Nos. 5,290,684, 5,624,840, 5,837,234, 5,853,717, and 5,935,849. Suspension-type bioartificial livers comprise cells suspended in plate dialysers, microencapsulated in a suitable substrate, or attached to microcarrier beads coated with extracellular matrix. Alternatively, hepatocytes can be placed on a solid support in a packed bed, in a multiplate flat bed, on a microchannel screen, or surrounding hollow fiber capillaries. The device has an inlet and outlet through which the subject's blood is passed, and sometimes a separate set of ports for supplying nutrients to the cells.

Microtissues are prepared according to the methods described earlier, and then plated into the device on a suitable substrate, such as a matrix of MATRIGEL® or collagen. The efficacy of the device can be assessed by comparing the composition of blood in the afferent channel with that in the efferent channel-in terms of metabolites removed from the afferent flow, and newly synthesized proteins in the efferent flow.

Devices of this kind can be used to detoxify a fluid such as blood, wherein the fluid comes into contact with the hepatocytes provided in certain aspects of the present disclosure under conditions that permit the cell to remove or modify a toxin in the fluid. The detoxification will involve removing or altering at least one ligand, metabolite, or other compound (either natural or synthetic) that is usually processed by the liver. Such compounds include but are not limited to bilirubin, bile acids, urea, heme, lipoprotein, carbohydrates, transferrin, hemopexin, asialoglycoproteins, hormones like insulin and glucagon, and a variety of small molecule drugs. The device can also be used to enrich the efferent fluid with synthesized proteins such as albumin, acute phase reactants, and unloaded carrier proteins. The device can be optimized so that a variety of these functions is performed, thereby restoring as many hepatic functions as are needed. In the context of therapeutic care, the device processes blood flowing from a patient in hepatocyte failure, and then the blood is returned to the patient.

### D. Distribution for Commercial, Therapeutic, and Research Purposes

In some embodiments, a reagent system is provided that includes microtissue that exists at any time during manufacture, distribution or use. The kits may comprise any combination of the microtissue described in the present disclosure in combination with undifferentiated pluripotent stem cells or other differentiated cell types, often sharing the same genome. Each cell type may be packaged together, or in separate containers in the same facility, or at different locations, at the same or different times, under control of the same entity or different entities sharing a business relationship. Pharmaceutical compositions may optionally be packaged in a suitable container with written instructions for a desired purpose, such as the mechanistic toxicology.

In some embodiments, a kit that can include, for example, one or more media and components for the production of microtissue is provided. The reagent system may be packaged either in aqueous media or in lyophilized form, where appropriate. The container means of the kits will generally include at least one vial, test tube, flask, bottle, syringe or other container means, into which a component may be placed, and preferably, suitably aliquoted. Where there is more than one component in the kit, the kit also will generally contain a second, third or other additional container into which the additional components may be separately placed. However, various combinations of components may be comprised in a vial. The components of the kit may be provided as dried powder(s). When reagents and/or components are provided as a dry powder, the powder can be reconstituted by the addition of a suitable solvent. It is envisioned that the solvent may also be provided in another container means. The kits of the present disclosure also will typically include a means for containing the kit component(s) in close confinement for commercial sale. Such containers may include injection or blow molded plastic containers into which the desired vials are retained. The kit can also include instructions for use, such as in printed or electronic format, such as digital format.

### IV. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result.

### Example 1 - Evaluation of Parameters for Microtissue Production

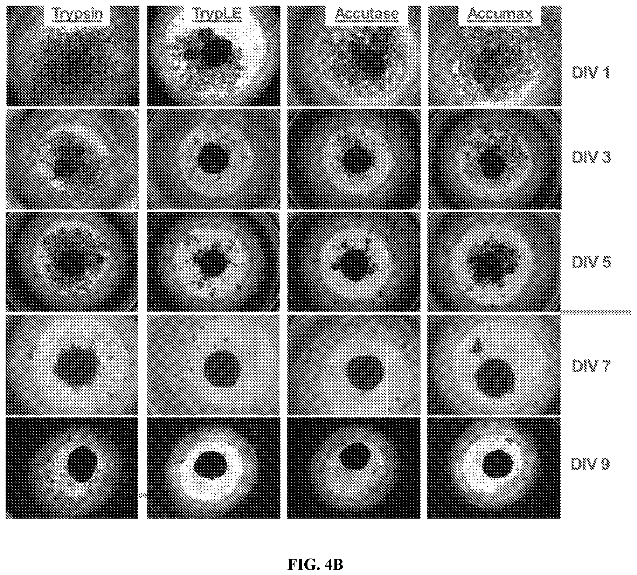

To produce functional, 3-D microtissue from stem cell-derived hepatocytes such as iCell® Hepatocytes (Cellular Dynamics International, Inc.), several parameters were evaluated to optimize microtissue formation. First, it was determined whether the iCell® Hepatocytes could be thawed and directly cultured in a 3-D culture or whether they should be pre-plated in a traditional 2-D culture to allow the cells to recover from cryopreservation. Accordingly, an aliquot of iCell® Hepatocytes 2.0 was thawed and resuspended in Plating Medium (Table 2) to prepare a cell suspension of about 1 million cells per mL and then seeded into a Collagen type I coated 6-well cell culture plate. Once a confluent culture was obtained (FIG. 2A), the cells were detached by removing the medium from the cells, washing the cells once with 3 mL/well of PBS (without calcium or magnesium chloride), and applying 1 mL/well of ACCUTASE®, Trypsin, TRYPLE™ or ACCUMAX™. The cell suspensions were then supplemented with 20% GELTREX® and re-plated in an ultra-low attachment plate. It was observed that pre-plating in a 2-D culture format increased the microtissue formation. In addition, the dissociation agent used to dissociate the hepatocytes into a cell suspension was found to have an effect on the production of microtissue. After about 48-72 hours, it was observed that cells that had been dissociated with gentler dissociation reagents, such as ACCUTASE® or TRYPLE™, formed microtissue more effectively than those dissociated with Trypsin (FIG. 4B).

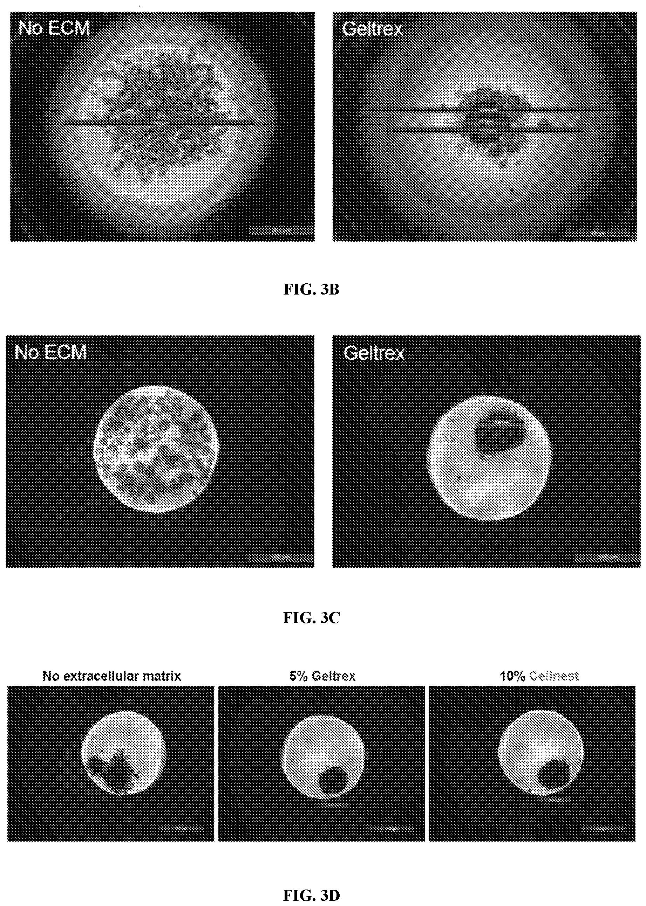

The next parameter that was evaluated was the supplementation of the iCell® Hepatocytes with extracellular matrix proteins or peptides. A low percentage of extracellular matrix (ECM) (*e.g.,* 5, 10 or 20%) was added to a cell suspension of iCell® Hepatocytes so that the cell suspension did not gel (FIG. 3D). It was found that extracellular matrix supplementation at low percentages promotes robust microtissue formation from stem cell-derived human liver cells by coating the cells and promoting cell-cell adhesion (FIG. 3B, 3D), while iCell® Hepatocytes with no ECM supplementation failed to efficiently form microtissue. To determine the effect of the percentage of extracellular matrix supplementation, iCell® Hepatocytes were supplemented with GELTREX® at a final concentration (*i.e.,* volume of matrix/volume of cell suspension) of 0.625, 1.25, 2.5, 5, 10 and 20% volume and plated in Corning round-bottom ULA plates at a cell density of 20, 40, 60 or 80 thousand cells per well (FIG. 3A). It was observed that microtissues formed at all cell densities when supplemented with 20% GELTREX®; however, even supplementation with as low as 5% GELTREX® or 10% CELLNEST™ was able to promote microtissue formation.

Another factor that was evaluated in the formation of microtissue was the use of a low attachment culture plate. To characterize the effect of the plate on microtissue formation, iCell® Hepatocytes with or without 20% GELTREX® supplementation were plated in Corning round-bottom ULA plates in a 384-well format (FIG. 3B; images taken 3 days post-thaw) or InSphero GravityTRAP ULA plates in a 96-well format (FIG. 3C; images taken 2 days post-thaw). Culture of the iCell® Hepatocytes supplemented with 20% GELTREX® in both of the ultra-low attachment plates was observed to produce microtissue.

In addition, other ECM proteins also promoted microtissue formation of ICELL® Hepatocytes in GravityTRAP ULA plates. Microtissue formation was observed when supplemented with 0.15 mg/mL collagen type 1, 0.15mg/mL collagen type 3, 0.15 mg/mL collagen type 4, 0.05mg/mL fibronectin, 0.03mg/mL laminin, 0.03mg/mL vitronectin, and 5mg/mL gelatin (FIG. 3E).

The final parameter for microtissue formation that was evaluated was the number of cells that are seeded in each well. The iCell® Hepatocytes were thawed and cultured for about 9 days in 2-D culture before dissociation and re-plating in an ultralow attachment plate at varying cell numbers, such as 400, 600, 2000, 4500, 10000, and 23000. Surprisingly, it was found that the microtissue size could be tuned to a desired size by modulating the number of cells seeded per well (FIG. 5). For example, seeding 2,500 cells per well resulted in a microtissue with a diameter of about 250 µm (FIG. 6B) and seeding 15,000 cells per well produced a microtissue of about 500 - 600 µm in diameter (FIG. 5A & 5B), depending on the extracellular matrix supplementation used and the timepoint when the measurement was taken after the 3-D culture was initiated. Thus, it was determined that 3-D microtissue can be efficiently produced by supplementing a cell suspension of PSC-derived hepatocytes with a low percentage of ECM proteins or recombinant peptides and plating in an ultra-low attachment culture plate. In addition, pre-plating of the cryopreserved cells in a 2-D culture format and subsequent dissociation with ACCUTASE® increases the production of 3-D microtissue, although it is not critical or required.

### Example 2 - Generating 3-D Microtissues from PSC-Derived Hepatocytes

The evaluation of parameters in microtissue production in Example 1 led to an optimized method of producing 3-D microtissue from PSC-derived hepatocytes. First, a cell suspension of stem-cell derived hepatocytes was obtained by thawing an aliquot of iCell® Hepatocytes according to the iCell® Hepatocytes 2.0 User Guide. The thawed hepatocytes were resuspended in Plating Medium (Table 2) to prepare a cell suspension of about 1 million cells per mL. To allow the hepatocytes to recover from thawing, the cell suspension was then seeded into a Collagen type I coated 6-well cell culture plate at a cell density of about 3 million cells per well. The hepatocytes were then cultured at 37°C, 5% CO₂ for about 3-4 hours. Following the incubation, the 6-well cell culture plate was shaken in diagonal planes 4 times to remove dead cells and debris. The Plating Medium was then aspirated from the plate using a pipettor and replaced with 3 mL/well of room temperature Plating Medium. The iCell® Hepatocytes 2.0 were cultured for four days and fed fresh Plating Medium each day. On day 5, the iCell® Hepatocytes were then cultured in Maintenance Medium for about 48 hours.

**Table 2: Plating Medium**

| **Component** | **Amount (mL)** | **Final Concentration** |
|---|---|---|
| RPMI | 72 | 96% |
| B27 Supplement, 50x | 1.5 | 1x |
| Oncostatin M, 10 ug/mL | 0.15 | 20 ng/ mL |
| Dexamethasone, 5 mM | 0.0015 | 0.1 uM |
| Gentamicin | 0.0375 | 25 ug/ mL |
| iCell® Hepatocytes 2.0 Medium Supplement (LCAA) | 1.5 | 1x |

**Table 3: Maintenance Medium**

| **Component** | **Amount (mL)** | **Final Concentration** |
|---|---|---|
| RPMI | 72 | 96% |
| B27 Supplement, 50x | 1.5 | 1x |
| Dexamethasone, 5 mM | 0.0015 | 0.1 uM |
| Gentamicin | 0.0375 | 25 ug/ mL |
| ICELL® Hepatocytes 2.0 Medium Supplement (LCAA) | 1.5 | 1x |

Once a confluent culture was obtained (FIG. 2A), the cells were detached by removing the medium from the cells, washing the cells once with 3 mL/well of PBS (without calcium or magnesium chloride), and applying 1 mL/well of ACCUTASE® cell detachment reagent. The cells were incubated about 2-4 minutes at room temperature until the cells just began to come off the plate. Next, 2 mL/well of Maintenance Medium (Table 3) or Supplemented Williams' E Medium (SWE; Williams' E Medium supplemented with Hepatocyte Maintenance Supplement Cocktail B, a cocktail solution of penicillin-streptomycin, ITS+ [insulin, transferrin, selenium complex, BSA, and linoleic acid], GlutaMAX™, and HEPES) were added to dilute the dissociation reagent and gently pipetted up and down several times around the well to ensure the cells were detached and to generate a cell suspension with small clusters of cells. After the cell suspension was transferred to a 15 mL tube, the well was rinsed with 3 mL/well of Maintenance Medium or SWE and also added to the 15 mL tube.

The cells were gently pelleted by centrifugation and then resuspended in Maintenance Medium or SWE supplemented with GELTREX® (*i.e,* 8 mL of Maintenance Medium or SWE and 2 mL of ready-to-use GELTREX® matrix) or CELLNEST™ (*i.e.,* 8 mL of Maintenance Medium or SWE and 4 mL of CELLNEST™). The cell suspension supplemented with either GELTREX® or CELLNEST™ was seeded in a GravityTRAP™ ULA 96-well spheroid plate at a volume of 70 µL/well. The spheroid plate was centrifuged to remove any air bubbles and settle the cells to the bottom of the wells, at 200 × g for 3 minutes at room temperature. The iCell® Hepatocytes were cultured for about 48 hours to obtain functional microtissue (FIG. 2B).

### Example 3 - Characterization of Microtissue Function

The viability of the microtissue produced in Example 2 was determined by the quantitation of ATP, which is a marker for the presence of metabolically active cells. The microtissue was maintained by replacing the medium with fresh Maintenance Medium every 2 days. To remove the medium the pipette tip was placed at an angle along the side of the well but not touching the well bottom and the used medium was carefully removed at low pipetting speed (*e.g.,* <30 µL/sec) so that a minimal volume of about 5-7 µL remained in the bottom of the well. 70 µL of Maintenance Medium warmed to room temperature was then added to each well and the plate was centrifuged.

The CELLTITER-GLO® 3-D Reagent was added to spheroids produced from iCell® Hepatocytes cultured in a 2-D format for 10 days after thawing and supplemented with 20% final volume of GELTREX® and the resultant luminescent signal (RLU) was measured (FIG. 7A). It was observed that there is a correlation between the microtissue size and the levels of ATP, indicating that the microtissue is indeed viable.

To characterize the functionality of the microtissue, the cytochrome P540 activity was determined as drug metabolism is a major function of the liver. To measure the activities of the CYP enzymes in the microtissue, day 6 spheroids supplemented with 20% final volume of GELTREX® were treated with about 0.1 to 100 µM of Omeprazole or diluent for about 24 hours. The P450-GLO™ CYP1A2 substrate was added to the culture plate followed by Luciferin Detection Reagent and subsequent measurement of luminescence on a luminometer (FIG. 7B, left). It was observed that Omeprazole induced CYP1A2 activity in the microtissue by about 2.5 fold. In addition, day 9 spheroids were treated with Rifampicin for 96 hours and the P450-GLO ™ CYP3A4 Assay showed an almost 3.2 fold induction of activity (FIG. 7B, right).

The CYP3A4 activity induced by Rifampicin in the microtissue was then compared to the CYP3A4 activity of iCell® Hepatocytes cultured in a 2-D format for 14 days after thawing (FIG. 7C). Both cultures were treated with about 0.1 µM to about 10 µM Rifampicin for about 72 hours before the addition of the reagent. While the 2-D culture showed about 1.7-fold induction of CYP3A4 activity, the 3-D culture had an almost 3.2-fold induction of activity. Thus, the microtissues have significantly improved function as compared to traditional 2-D cultures.

**Table 4: Materials**

| **Item** | **Supplier** | **Catalog Number** |
|---|---|---|
| iCell® Hepatocytes 2.0 Kit | Cellular Dynamics International | PHC-100-020-001 |
| CORNING® BIOCOAT™ Collagen I 6 Well Clear Flat Bottom TC-Treated Multiwell Plate (2-D format) | Corning | 354400 |
| STEMPRO® ACCUTASE® Cell Dissociation Reagent | ThermoFisher Scientific | A11105-01 |
| Dulbecco's Phosphate Buffered Saline (DPBS), no calcium chloride, no magnesium chloride | ThermoFisher Scientific | 14190-144 |
| GELTREX® hESC-qualified Ready-To-Use Reduced Growth Factor Basement Membrane Matrix | ThermoFisher Scientific | A15696-01 |
| CELLNEST™ recombinant peptide based on collagen type I, 0.1% solution | FUJIFILM | |
| Collagen Type I, Bovine Skin, Pepsin-Solubilized (3mg/mL) | Nippi | PSC-1-100-100 |
| Collagen Type III, Bovine Skin, Pepsin-Solubilized (3mg/mL) | Nippi | PSC-3-100-100 |
| Collagen Type IV, Bovine Lens, Acid Soluble (0.5mg/mL) | Nippi | PSC-4-104-01 |
| Fibronectin, from Bovine Plasma | Wako | 068-05703 |
| Laminin solution, from Mouse EHS Tumor | Wako | 120-05751 |
| Vitronectin, from Human Plasma | Wako | 220-02281 |
| Low Endotoxin Gelatin from Porcine Skin (Mw : 60,000, High Grade) | Nippi | APAT |
| InSphero GravityTRAP™ ULA Plate (3-D format) | PerkinElmer | ISP-09-001 |

### Example 4 - Optimization of Microtissue Formation

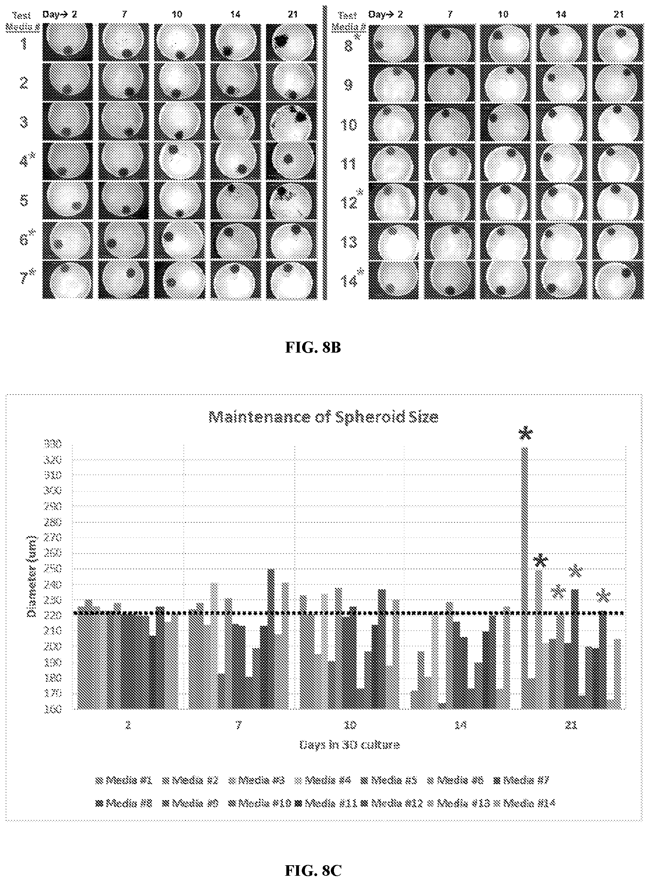

Additional development of the microtissue formation evaluated other parameters for the optimal production of the microtissue. The first parameter tested was which media formulation best supports the maintenance of the morphological and functional integrity of the microtissue in 3-D culture (FIG. 8A). A 2-D culture of iCell® Hepatocytes 2.0 was used as source material to form spheroids in 96-well format (InSphero GravityTRAP ULA plate) using about 1,000 cells/well in Supplemented Williams' E (SWE) medium with 10% GELTREX®. The cells were cultured in 2-D format for 5 days post-thaw prior to detaching the cells, resuspending them in SWE and re-plating into 3-D format. On Day 1 of the 3-D culture, the microtissue formation medium was replaced with freshly prepared microtissue test Maintenance Medium conditions (Table 5). The 3-D cultures were fed with the designated test media every 2-3 days throughout the 21-day time course. The 3-D cultures were assayed at several time-points up to Day 21 for maintenance of several key parameters including morphology, size, viability, and CYP3A4 function. It was observed that several of the Maintenance Media compositions including 4, 6, 7, 8, 12, and 14 had improved spheroid morphology as compared to other conditions (FIG. 8B). Evaluation of spheroid size maintenance (FIG. 8C-D) showed that conditions 6, 8, and 12 resulted in the preferred diameter size. Cell viability measurements showed that conditions 12 and 14 maintained ATP levels out to Day 21 (FIGS. 8E-8F). Finally, CYP3A4 levels showed that the conditions 4, 6, 7, 8, and 12 maintained basal CYP3A4 activity at a consistent level throughout the time course (FIGS. 8G-8H).

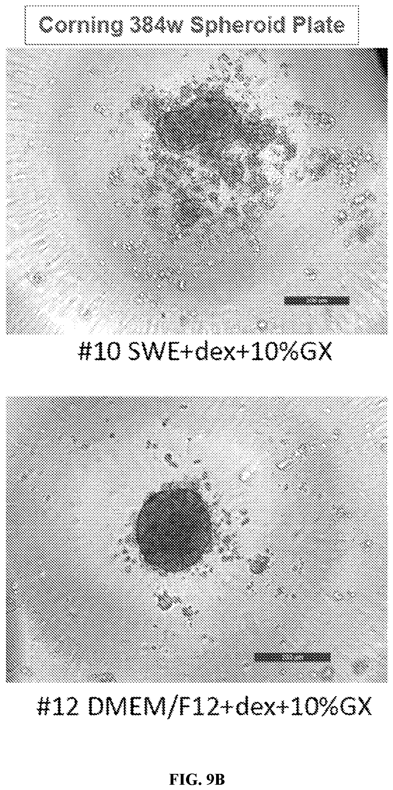

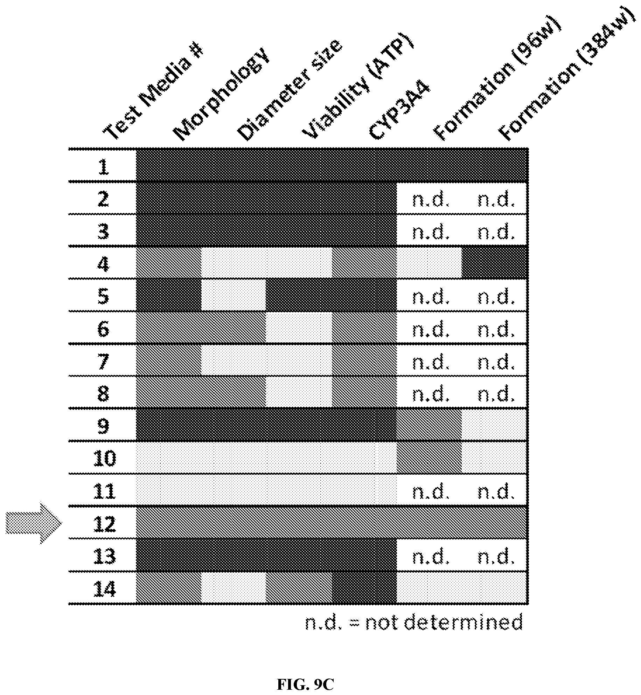

Further, spheroid formation was tested in 96-well or 384-well formats (InSphero GravityTRAP ULA plate or the Corning round-bottom ULA plate) using about 1,750 to 2,000 cells/well in Test Medias 1, 9, and 12 with 10% GELTREX®. The GravityTRAP plate was found to be more efficient at generating spheroids by 3-D culture day 1 than the Corning spheroid plate (FIG. 9A). In addition, Test Media #12 yielded more efficient spheroid formation than the previous SWE spheroid formation media condition (also known as Test Media #10) in the stringent 384-well plate format (FIG. 9B). In summary, Test Media #12 best met all the test criteria as demonstrated by enabling efficient spheroid formation in both 96-well and 384-well formats and maintaining spheroid longevity (size, morphology and viability maintained for >21 days) and CYP3A4 function (FIG. 9C). Test Media #12 is also referred to as Maintenance Medium 2 (MM2) or Spheroid Maintenance Medium and is comprised of 98% DMEM/F-12 Medium, IX B27 Supplement, 0.1 µM dexamethasone, and 25 µg/mL Gentamicin (Table 6).

**Table 5: Test Media Formulations**

| | | **1** | **2** | **3** | **4** | **5** | **6** | 7 | **8** | **9** | **10** | **11** | **12** | **13** | **14** |
|---|---|---|---|---|---|---|---|---|---|---|---|---|---|---|---|
| | Buffered | No | No | Yes | Yes | Yes | Yes | Yes | Yes | No | No | Yes | Yes | Yes | Yes |
| | LCAA (L-carnitine ascorbic acid) | Yes | No | Yes | No | Yes | No | No | No | No | No | No | No | No | No |
| | Phenol red | Yes | Yes | Yes | Yes | Yes | Yes | No | Yes | No | No | No | No | No | Yes |
| | Na Pyruvate | No | No | No | No | Yes | Yes | Yes | Yes | Ye s | Yes | Yes | Yes | Yes | Yes |

| **Catalog No.** | **Component** | **mL** | **mL** | **mL** | **mL** | **mL** | **mL** | **mL** | **mL** | **m** L | **mL** | **mL** | **mL :** | **mL** | **mL** |
|---|---|---|---|---|---|---|---|---|---|---|---|---|---|---|---|
| 11875-085 | RPMI 1640 | 48 | 49 | 47 | 48 | 47 | 48 | - | - | - | - | - | - | - | |
| 32404-014 | RPMI 1640, no glutamine, no phenol red | - | - | - | - | - | - | 47 | - | - | - | - | - | - | - |
| 12633-012 | Advanced RPMI 1640 | - | - | - | - | - | - | - | 47.5 | - | - | - | - | - | - |
| 11039-021 | DMEM/F12, HEPES, no phenol red | - | - | - | - | - | - | - | - | - | - | - | 49 | - | - |
| 21083-027 | Leibovitz's L-15, no phenol red | - | - | - | - | - | - | - | - | - | - | - | - | 48 | - |
| 11220-035 | Waymouth's | - | - | - | - | - | - | - | - | - | - | - | - | - | 47.5 |
| A12176-01 | Williams' Medium E | - | - | - | - | - | - | - | - | 48 | 48 | 47.5 | - | - | - |
| CM4000 | Cocktail B (pen-strep, ITS+, GlutaMAX, and HEPES) | - | - | - | - | - | - | - | - | 2 | 2 | - | - | - | - |
| 17504-044 | B27 | 1 | 1 | 1 | 1 | 1 | 1 | 1 | 1 | - | - | 1 | 1 | 1 | 1 |
| HCS-100-021-001 | iCell Hepatocytes 2.0 Medium Supplement | 1 | - | 1 | - | 1 | - | - | - | - | - | - | - | - | - |
| 15750-060 | Gentamicin | 0.02 5 | 0.02 5 | 0.02 5 | 0.02 5 | 0.02 5 | 0.02 5 | 0.02 5 | 0.02 5 | - | - | 0.02 5 | 0.02 5 | 0.02 5 | 0.02 5 |
| ICN19456125 | Dexamethasone (5 mM stock in DMSO), 0.1 µM final | 0.00 1 | 0.00 1 | 0.00 1 | 0.00 1 | 0.00 1 | 0.00 1 | 0.00 1 | 0.00 1 | - | 0.00 1 | 0.00 1 | 0.00 1 | 0.00 1 | 0.00 1 |
| 15630-080 | HEPES (1 M stock), 20 mM final concentration | - | - | 1 | 1 | 1 | 1 | 1 | 1 | - | - | 1 | - | 1 | 1 |
| 35050-061 | GlutaMAX-I (100X) | - | - | - | - | - | - | 0.5 | 0.5 | - | - | 0.5 | - | - | - |
| 11360-070 | Sodium Pyruvate (100X) | - | - | - | - | 0.5 | 0.5 | 0.5 | - | - | - | - | - | - | 0.5 |

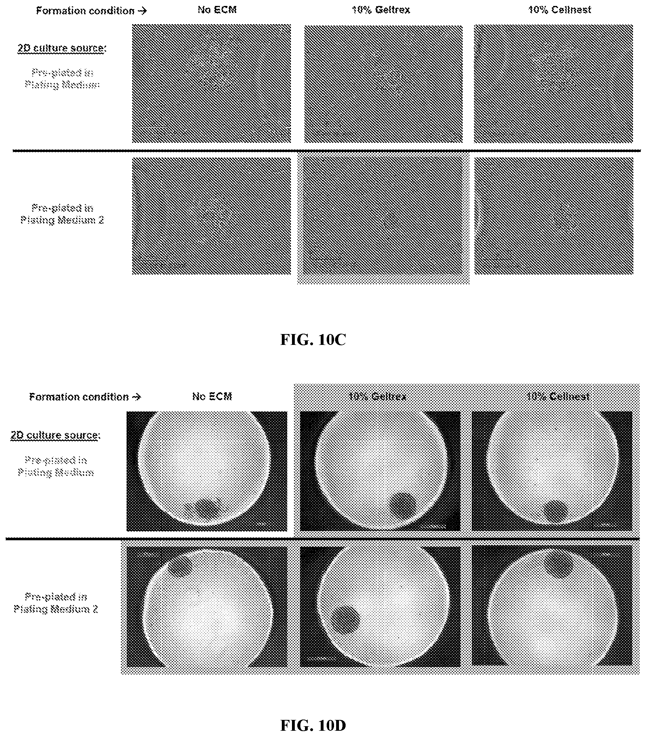

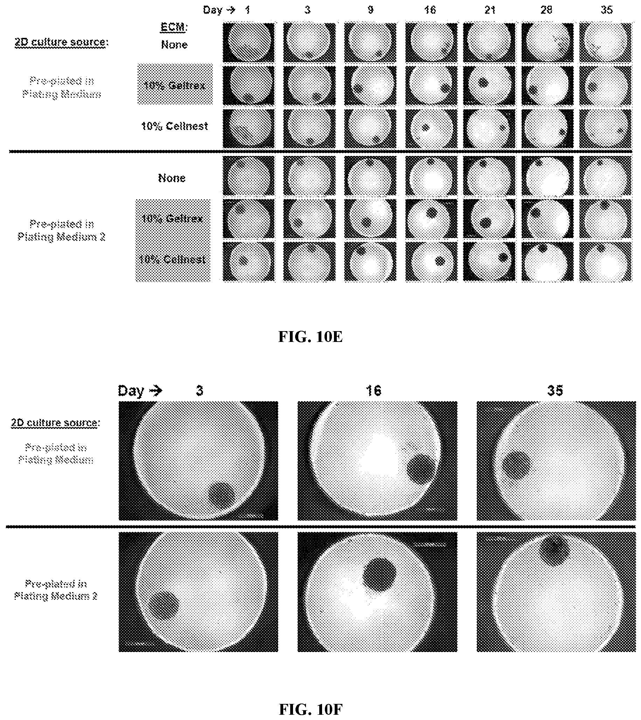

Next, the 2-D plating media formulation was evaluated for its ability to prepare the pre-plated cells for robust spheroid formation and longevity (FIG. 10A). iCell® Hepatocytes were plated into the 2-D format onto collagen I-coated plates using either the previously determined iCell® Hepatocytes 2.0 Plating Medium formulation (Table 2) or a new formulation referred to as Plating Medium 2 (PM2), which was comprised of MM2 media supplemented with Oncostatin M at 10 µg/mL (Table 6). After 5 days in culture, the cells were detached and resuspended in MM2 media with ECM supplementation comprising either 10% GELTREX® or 10% CELLNEST™ and replated in the 3-D format. The cells were then evaluated for their ability to efficiently form spheroids in different ultra-low attachment plates as well as their ability to maintain spheroid size and morphology for at least a month. Cells that had been pre-plated in PM2 exhibited a higher propensity for robust spheroid formation when plated into 96-well Corning round-bottom ULA plates: spheroids formed when supplemented with either 10% GELTREX® or 10% CELLNEST™ (FIG. 10B). When using the 384-well Corning round-bottom ULA plates, spheroids formed best with 10% GELTREX® from cells pre-plated in PM2 (FIG. 10C). In the GravityTRAP 96-well ULA plates, spheroids formed from cells pre-plated in both Plating Media formulations and then re-plated in MM2 supplemented with GELTREX® or CELLNEST™ (FIG. 10D). The longevity testing showed that spheroids generated from cells pre-plated in Plating Medium could maintain their morphology at Day 35 only when GELTREX® was included during the spheroid formation step. In contrast, cells pre-plated in PM2 exhibited increase ability to maintain spheroid morphology when either GELTREX® or CELLNEST™ was included in the spheroid formation step (FIG. 10E). FIG. 10F provides a close-up view of spheroid size and intactness being maintained out to 35 days for cells pre-plated in either Plating Medium and then supplemented with GELTREX® during the spheroid formation step. Functional testing was performed in which basal CYP3A4 activity was measured up to Day 35, showing that cells that had been pre-plated in PM2 prior to preparing spheroids in MM2 with either GELTREX® or CELLNEST™ best maintained their CYP3A4 activity (FIG. 10G). Thus, PM2 (also referred to as Plating Medium 2 or optimized 2-D Plating Medium) was found to be more suitable for preparing 2-D cultures for robust spheroid formation and longevity.

**Table 6: Optimized 2-D Plating Medium and Spheroid Maintenance Medium**

| | **2-D Plating Medium (Plating Medium 2)** | **Spheroid Maintenance Medium (Maintenance Medium 2)** | |
|---|---|---|---|
| **Component** | **Amount (mL)** | **Amount (mL)** | **Final Concentration** |
| DMEM/F-12 Medium without phenol red | 98 | 98 | 98% |
| B27 Supplement, 50x | 2 | 2 | 1x |
| Dexamethasone, 5 mM | 0.002 | 0.002 | 0.1 uM |
| Gentamicin | 0.05 | 0.05 | 25 ug/ mL |
| Oncostatin M, 10 ug/ mL | 0.2 | not included | 20 ng/ mL |

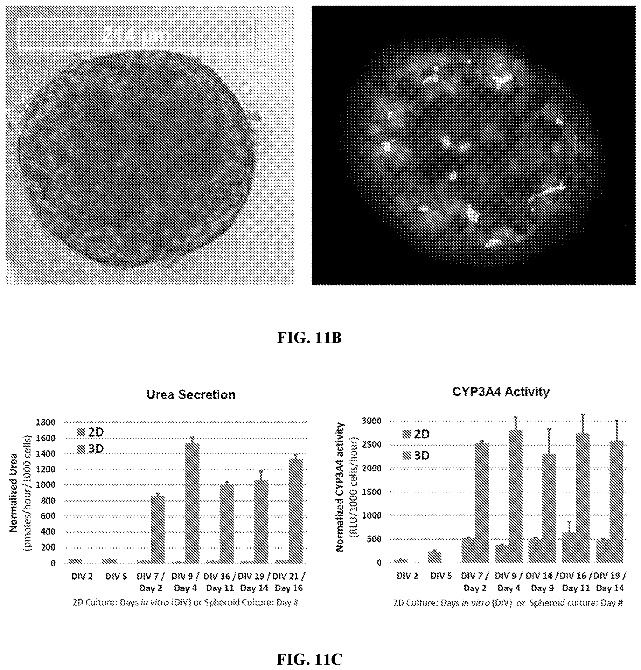

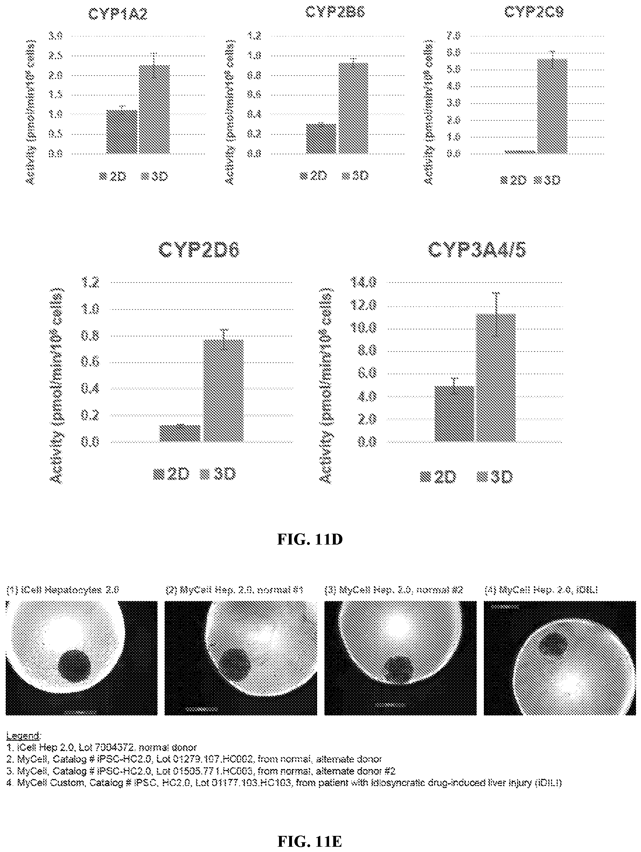

The Spheroid Maintenance Medium (MM2) was used during spheroid formation to confirm that spheroid formation can be achieved in different plate types and formats (FIG. 11A). The spheroids were found to have the ability to form bile canaliculi (FIG. 11B). The spheroids also exhibit elevated function as measured by urea secretion and basal CYP3A4 activity (P450-GLO assay) over time in 2-D and 3-D cultures of iCell Hepatocytes (FIG. 11C). To further characterize Cytochrome P450 enzymes activity, the activities of CYP1A2, CYP2B6, CYP2C9, CYP2-D6, and CRP3A4/5 were determined with probe substrates phenacetin, bupropion, diclofenac, dextromethorphan, and midazolam, respectively. Optimal reaction times of 2 h (2-D format) or 4h (3-D format) were used and the samples were analyzed by liquid chromatography paired with mass spectrometry (LC-MS/MS) (FIG. 11D). All enzymes activities were found to be increased in the 3-D cultures.

3-D liver spheroid formation was also tested from different iPSC donors (FIG. 11E), and it was found that 3-D liver spheroid formation can be achieved using hepatocytes generated from different iPSC donors. Next, the spheroids prepared from MyCell Hepatocytes 2.0 were tested for CYP3A4 induction at Day 14. It was observed that Rifampicin caused an about 3.3-fold induction in CYP3A4 activity in the spheroids versus a 1.6-fold induction in the 2-D culture (FIG. 11F), again confirming increased functionality when iPSC-derived hepatocytes are cultured in 3-D formats.

Next, cultures of iCell Hepatocytes in 2-D and 3-D formats were treated with select compounds including Diclofenac, Mitomycin C, and Tolcapone for varying times to assess hepatotoxicity responses. Increased sensitivity to compounds was observed with longer exposure times. The 3-D cultures exhibited different functional hepatotoxicity responses than traditional 2-D cultures (FIG. 12A).

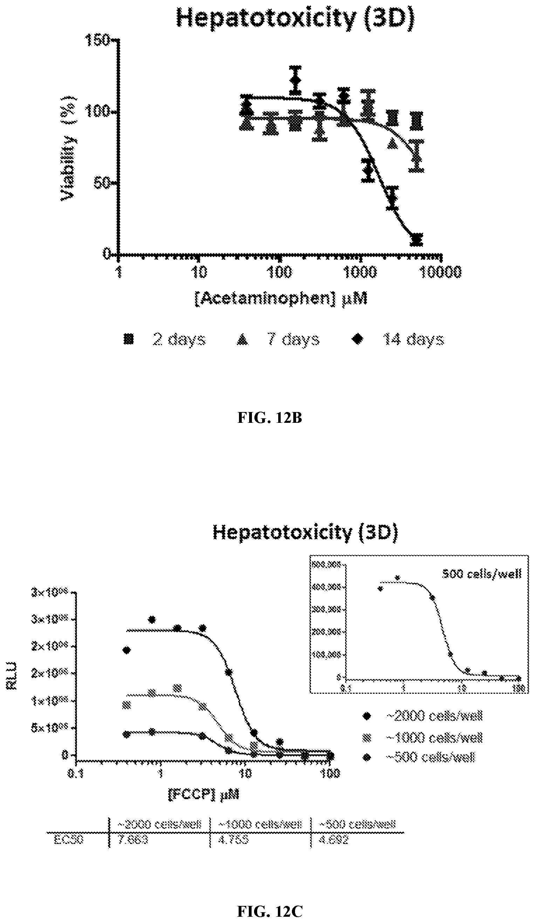

To assess whether the liver spheroids can be used to model drug-induced liver injury, 3-D cultures of iCell Hepatocytes were prepared and treated with acetaminophen for up to 14 days (FIG. 12B). Whereas no hepatotoxicity effects were observed for the acute, 48 h treatment time, longer exposure led to increased sensitivity towards high concentrations of the drug, confirming the suitability of these spheroid cultures for modeling drug-induced livery injury effects over time. In addition, assay miniaturization was tested by preparing 3-D cultures in a 96-well format using relatively low amounts of cells, such as 500 to 2,000 cells/well (versus about 30,000 cells/well required for the 96-well 2-D format). The cells were treated with the toxic compound Carbonyl cyanide-4-(trifluoromethoxy) phenylhydrazone (FCCP) and ATP determination (cell viability indicator) was performed using the Celltiter-GLO 3-D reagent (FIG. 12C). Excellent signal-to-noise was obtained even with spheroids as small as 500 cells/well. Thus, the 3-D spheroids enable hepatotoxicity miniaturization.

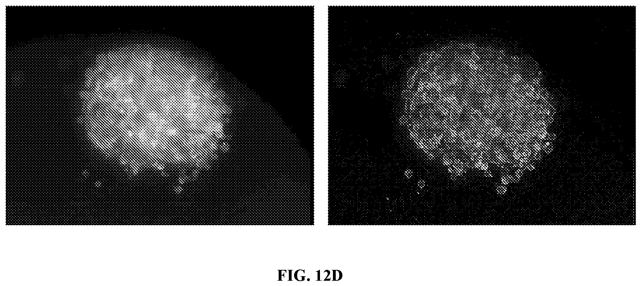

Finally, it was tested whether 3-D spheroid co-cultures can be formed by combining cell types. iCell Hepatocytes and iCell Macrophages were co-cultured together to generate human liver-like microtissue. Prior to mixing the two cell types together to prepare a spheroid co-culture, iCell Macrophages were stained with CellTracker Red and iCell Hepatocytes were stained with CellTracker Green (FIG. 12D). It was observed that the co-cultures can be formed by combining the cell types.

All of the methods disclosed and claimed herein can be made and executed without undue experimentation in light of the present disclosure.

### REFERENCES

Amit et al., Dev. Bio., 227:271-278, 2000.
Braun et al., Nature Med., 6:320, 2000.
Chen et al., Nature Methods, 8:424-429, 2011.
Clayton et al., Liver Int, 25(2)389-402, 2005.
Gripon et al., Proc. Natl. Acad. Sci. USA 99:15655-15660, 2002.
Grompe et al., Sem. Liver Dis., 19:7, 1999.
International Patent Publication No. PCT/US2015/026583
International Patent Publication No. PCT/US2015/026583
International Patent Publication No. WO 2007/069666
International Patent Publication No. WO 2007/069666
International Patent Publication No. WO 98/30679
International Patent Publication No. WO2008103041
Klimanskaya et al., Lancet., 365(9471):1636-41, 2005.
Kobayashi et al., Science, 287:1258, 2000.
LeCluyse et al., Methods Mol Biol., 290:207-29. 2005.
Lieber et al., Proc. Natl. Acad. Sci. U.S.A., 92:6210, 1995.
Ludwig et al., Nat. Biotechnol., 24:185-187, 2006b.
Ludwig et al., Nat. Methods, 3:637-646, 2006a.
Macdonald et al., In: Cell Encapsulation Technology and Therapeutics, Kuhtreiber et al., eds., Birkhauser, Boston Mass., pp. 252-286, 1999.
Mignon et al., Nature Med., 4:1185, 1998.
Ohashi et al., Nature Med., 6:327, 2000.
Overturf et al., Human Gene Ther., 9:295, 1998.
Parent et al., Gastroenterology 126:1147-1156, 2004.
Peeters et al., Hepatology, 25:884, 1997.
Reubinoff et al., Nat. Biotechnol., 18:399-404, 2000.
Rhim et al., Proc. Natl. Acad. Sci. U.S.A., 92:4942, 1995.
Rudolph et al., Science, 287:1253, 2000.
Smith, In: Origins and Properties of Mouse Embryonic Stem Cells, Annu. Rev. Cell. Dev. Biol., 2000.
Swifta et al., Drug Metab Rev., 42(3):446-471, 2010.
Takahashi et al., Cell, 126:663-676, 2007.
Thomson and Marshall, Curr. Top. Dev. Biol., 38:133-165, 1998.
Thomson and Odorico, J. Trends. Biotechnol., 18:53-57, 2000.
Thomson et al., Proc. Natl. Acad. Sci. U.S.A., 92:7844-7848, 1995.
Thomson et al., Science, 282:1145, 1998.
U.S. Patent No. 5,290,684
U.S. Patent No. 5,624,840
U.S. Patent No. 5,837,234
U.S. Patent No. 5,843,780
U.S. Patent No. 5,843,780
U.S. Patent No. 5,853,717
U.S. Patent No. 5,935,849
U.S. Patent No. 6,103,470
U.S. Patent No. 6,200,806
U.S. Patent No. 6,416,998
U.S. Patent No. 7,029,913
U.S. Patent No. 7,442,548
U.S. Patent No. 7,598,364
U.S. Patent No. 7,989,425
U.S. Patent No. 8,058,065
U.S. Patent No. 8,071,369
U.S. Patent No. 8,129,187
U.S. Patent No. 8,183,038
U.S. Patent No. 8,268,620
U.S. Patent No. 8,268,620
U.S. Patent No. 8,278,620
U.S. Patent No. 8,481,317
U.S. Patent No. 8,481,317
U.S. Patent No. 8,546,140
U.S. Patent No. 8,546,140
U.S. Patent No. 8,691,574
U.S. Patent No. 8,741,648
U.S. Patent No. 8,741,648
U.S. Patent No. 8,900,871
U.S. Patent No. 9,175,268
U.S. Patent Publication No. 2002/076747
U.S. Patent Publication No. 2003/0211603
U.S. Patent Publication No. 20090246875
U.S. Patent Publication No. 2010/0210014
U.S. Patent Publication No. 20120276636
U.S. Patent Publication No. 20120329157
U.S. Patent Publication No. 20130329157
U.S. Patent Publication No. 20140242595
U.S. Patent Publication No. 20140242595
U.S. Patent Publication No. 20140315304
Vickers, In: In vitro Methods in Pharmaceutical Research, Academic Press, 375-410, 1997.
Xu et al., Nat. Biotechnol., 19:971-974, 2001.
Yamanaka *et al.* 2006.
Ying et al., Cell, 115:281-292, 2003.
Yu et al., Science, 318:1917-1920, 2007.

## Claims

1. An *in vitro* method of producing microtissue from pluripotent stem cell (PSC)-derived hepatocytes comprising culturing a cell suspension of PSC-derived hepatocytes in the presence of one or more extracellular matrix proteins in a culture container having an essentially cell non-adhesive bottom to obtain microtissue,
wherein the culture container is an ultra-low attachment plate and
wherein the one or more extracellular matrix proteins are selected from the group consisting of laminin, collagen type IV, entactin, heparin sulfate proteoglycan, collagen type I, fibronectin, matrix extracellular phosphoglycoprotein (MEPE), nidogen-1, F-spondin, R-spondin, tenascin, testican, vitronectin, and decorin.

2. The method of claim 1, further comprising supplementing the cell suspension with one or more extracellular matrix proteins.

3. The method of claim 1, wherein the culture container does not comprise a scaffold or matrix overlay.

4. The method of claim 1, wherein the pluripotent stem cell is human induced pluripotent stem cell.

5. The method of claim 1, wherein the one or more extracellular matrix proteins is collagen type I recombinant peptide.

6. The method of claim 1, wherein the one or more extracellular matrix proteins is (i) a non-animal recombinant peptide (RCP) based on human collagen type I that is enriched with arginine-glycine-aspartate (RGD) sequences to enhance cell adhesion.

7. The method of claim 1, wherein the one or more extracellular matrix proteins are laminin, collagen type IV, entactin, and heparin sulfate proteoglycan.

8. The method of claim 1,
wherein the cell suspension of PSC-derived hepatocytes is obtained by:
(i) thawing cryopreserved PSC-derived hepatocytes;
(ii) culturing the PSC-derived hepatocytes in a two-dimensional (2-D) culture; and
(iii) dissociating the PSC-derived hepatocytes with a cell detachment reagent to obtain a cell suspension.

9. The method of claim 8, wherein culturing is in the presence of oncostatin M.

10. The method of claim 8, wherein the 2-D culture is on an adhesive surface, the adhesive surface is a matrix, and the matrix comprises at least one extracellular matrix protein.

11. The method of claim 10, wherein the at least one extracellular matrix protein is collagen, laminin, or fibronectin.

12. The method of claim 8, wherein the PSC-derived hepatocytes are not dissociated into essentially single cells.

13. The method of claim 8, wherein the dissociation does not disrupt clusters of cells.

## Patentansprüche

1. *In vitro*-Verfahren zum Produzieren von Mikrogewebe aus von pluripotenten Stammzellen (pluripotent stem cell; PSC) abgeleiteten Hepatozyten, umfassend das Kultivieren einer Zellsuspension aus von PSC abgeleiteten Hepatozyten in der Gegenwart eines oder mehrerer extrazelluläre-Matrix-Proteine in einem Kulturbehälter mit einem im Wesentlichen nicht-zelladhäsiven Boden, um Mikrogewebe zu erhalten,
wobei der Kulturbehälter eine Platte mit ultrageringer Anhaftung (ultra-low attachment plate) ist, und
wobei das eine oder die mehreren extrazelluläre-Matrix-Protein(e) aus der Gruppe ausgewählt ist/sind, bestehend aus Laminin, Kollagen Typ IV, Entactin, Heparinsulfatproteoglycan, Kollagen Typ I, Fibronektin, extrazelluläre-Matrix-Phosphoglycoprotein (matrix extracellular phosphoglycoprotein; MEPE), Nidogen-1, F-Spondin, R-Spondin, Tenascin, Testican, Vitronectin und Decorin.

2. Verfahren nach Anspruch 1, weiterhin umfassend das Supplementieren der Zellsuspension mit einem oder mehreren extrazelluläre-Matrix-Protein(en).

3. Verfahren nach Anspruch 1, wobei der Kulturbehälter keine Gerüst- oder Matrixauflage umfasst.

4. Verfahren nach Anspruch 1, wobei die pluripotente Stammzelle eine humane induzierte pluripotente Stammzelle ist.

5. Verfahren nach Anspruch 1, wobei das eine oder die mehreren extrazelluläre-Matrix-Protein(e) ein rekombinantes Peptid aus Kollagen Typ I ist/sind.

6. Verfahren nach Anspruch 1, wobei das eine oder die mehreren extrazelluläre-Matrix-Protein(e) (i) ein nichttierisches rekombinantes Peptid (recombinant peptide; RCP) auf Basis von humanem Kollagen Typ I ist, das mit Arginin-Glycin-Aspartat (RGD)-Sequenzen angereichert ist, um die Zelladhäsion zu verstärken.

7. Verfahren nach Anspruch 1, wobei das eine oder die mehreren extrazelluläre-Matrix-Protein(e) Laminin, Kollagen Typ IV, Entactin und Heparinsulfatproteoglycan ist/sind.

8. Verfahren nach Anspruch 1, wobei die Zellsuspension aus von PSC abgeleiteten Hepatozyten erhalten wird durch:
(i) Auftauen von kryokonservierten, von PSC abgeleiteten Hepatozyten;
(ii) Kultivieren der von PSC abgeleiteten Hepatozyten in einer zweidimensionalen (2-D) Kultur; und
(iii) Dissoziieren der von PSC abgeleiteten Hepatozyten mit einem Zellablösungsreagens, um eine Zellsuspension zu erhalten.

9. Verfahren nach Anspruch 8, wobei das Kultivieren in Gegenwart von Oncostatin M erfolgt.

10. Verfahren nach Anspruch 8, wobei die 2-D-Kultur auf einer adhäsiven Oberfläche erfolgt, die adhäsive Oberfläche eine Matrix ist und die Matrix wenigstens ein extrazelluläre-Matrix-Protein umfasst.

11. Verfahren nach Anspruch 10, wobei das wenigstens eine extrazelluläre-Matrix-Protein Kollagen, Laminin oder Fibronektin ist.

12. Verfahren nach Anspruch 8, wobei die von PSC abgeleiteten Hepatozyten nicht zu im Wesentlichen einzelnen Zellen dissoziiert sind.

13. Verfahren nach Anspruch 8, wobei die Dissoziation keine Cluster von Zellen stört.

## Revendications

1. Procédé *in vitro* de production de microtissu à partir d'hépatocytes dérivés de cellules souches pluripotentes (PSC) comprenant la mise en culture d'une suspension de cellules d'hépatocytes dérivés de PSC en présence d'une ou plusieurs protéines de matrice extracellulaire dans un récipient de culture ayant un fond essentiellement non adhésif pour les cellules pour obtenir un microtissu,
dans lequel le récipient de culture est une plaque de fixation ultra-faible et dans lequel les une ou plusieurs protéines de matrice extracellulaire sont choisies dans le groupe consistant en laminine, collagène type IV, entactine, héparine sulfate protéoglycane, collagène type I, fibronectine, phosphoglycoprotéine (MEPE) extracellulaire de matrice, nidogène-1, F-spondine, R-spondine, ténascine, testicane, vitronectine, et décorine.

2. Procédé selon la revendication 1, comprenant de plus la supplémentation de la suspension de cellules avec une ou plusieurs protéines de matrice extracellulaire.

3. Procédé selon la revendication 1, dans lequel le récipient de culture ne comprend pas une superposition de squelette ou matrice.

4. Procédé selon la revendication 1, dans lequel la cellule souche pluripotente est une cellule souche pluripotente humaine induite.

5. Procédé selon la revendication 1, dans lequel les une ou plusieurs protéines de matrice extracellulaire sont un peptide recombinant de collagène type I.

6. Procédé selon la revendication 1, dans lequel les une ou plusieurs protéines de matrice extracellulaire sont (i) un peptide recombinant non-animal (PRC) basé sur du collagène humain de type I qui est enrichi avec des séquences d'arginine-glycine-aspartate (RGD) pour promouvoir l'adhérence de cellule.

7. Procédé selon la revendication 1, dans lequel les une ou plusieurs protéines de matrice extracellulaire sont la laminine, le collagène type IV, l'entactine, et l'héparine sulfate protéoglycane.

8. Procédé selon la revendication 1,
dans lequel la suspension de cellules d'hépatocytes dérivés de PSC est obtenue par :
(i) congélation d'hépatocytes dérivés de PSC cryopréservées
(ii) mise en culture des hépatocytes dérivés de PSC dans une culture bi-dimensionnelle (2-D) ; et
(iii) dissociation des hépatocytes dérivés de PSC avec un réactif de détachement de cellule pour obtenir une suspension de cellules.

9. Procédé selon la revendication 8, dans lequel la mise en culture se fait en présence d'oncostatine M.

10. Procédé selon la revendication 8, dans lequel la culture 2-D se fait sur une surface adhésive, la surface adhésive est une matrice, et la matrice comprend au moins une protéine de matrice extracellulaire.

11. Procédé selon la revendication 10, dans lequel la au moins une protéine de matrice extracellulaire est le collagène, la laminine, ou fibronectine.

12. Procédé selon la revendication 8, dans lequel les hépatocytes dérivés de PSC ne sont pas dissociés en cellules essentiellement uniques.

13. Procédé selon la revendication 8, dans lequel la dissociation ne rompt pas les amas de cellules.
